# EUROPÄISCHE PATENTSCHRIFT

(11) **EP 3 924 702 B1**
(45) Veröffentlichungstag und Bekanntmachung des Hinweises auf die Patenterteilung: **17.12.2025**
(21) Anmeldenummer: 20704293.8
(22) Anmeldetag: 11.02.2020
(51) Int. Cl.: G01J 3/02, G01J 3/10, G01N 21/17, G01J 3/12

(54) **IR-EMITTER MIT MODULIERBAREM EMISSIONSGRAD AUF BASIS VON METAMATERIALIEN**
IR EMITTER WITH MODULAR EMISSIONS LEVEL BASED ON METAMATERIALS
ÉMETTEUR IR À DEGRÉ D'ÉMISSION MODULABLE À BASE DE MÉTAMATÉRIAUX

(30) Priorität: 12.02.2019 EP 19156588
(43) Veröffentlichungstag der Anmeldung: 22.12.2021
(73) Patentinhaber: Hahn-Schickard-Gesellschaft für angewandte Forschung e. V., 70569 Stuttgart (DE)
(72) Erfinder: BIESINGER, Daniel, 78054 Villingen-Schwenningen (DE); BITTNER, Achim, 74080 Heilbronn (DE)
(74) Vertreter: Hertin und Partner Rechts- und Patentanwälte PartG mbB
(86) Internationale Anmeldenummer: PCT/EP2020/053438
(87) Internationale Veröffentlichungsnummer: WO 2020/165150

(56) Entgegenhaltungen:
- EP-A1- 3 315 929
- WO-A1-2017/088071
- US-A1- 2012 170 114
- US-A1- 2017 288 125
- TAKUYA INOUE ET AL: "Realization of narrowband thermal emission with optical nanostructures", OPTICA, vol. 2, no. 1, 14 January 2015 (2015-01-14), pages 27, XP055330411, DOI: 10.1364/OPTICA.2.000027
- XINYU LIU ET AL: "Reconfigurable room temperature metamaterial infrared emitter", OPTICA, vol. 4, no. 4, 13 April 2017 (2017-04-13), pages 430, XP055418579, DOI: 10.1364/OPTICA.4.000430

## Beschreibung

Die Erfindung betrifft einen modulierbaren Infrarot-Emitter umfassend ein Heizelement, ein flächiges Grundelement, eine dielektrische Zwischenschicht und ein flächiges Deckelement, welches ein strukturiertes Metamaterial ist und einen Aktuator, wobei der Aktuator für eine Relativbewegung des Deckelementes und des Grundelementes zwischen einer ersten und zweiten Position konfiguriert ist, um die Intensität der Emission des Infrarot-Emitters zu modulieren. Die Erfindung betrifft weiterhin Herstellungsverfahren für den Infrarot-Emitter, Verfahren zur modulierten Emission von Infrarotrotstrahlung mittels des Infrarot-Emitters sowie bevorzugte Verwendungen des Infrarot-Emitters. Auch ein System umfassend den InfrarotEmitter und eine Steuereinrichtung zur Regulation des Aktuators sind bevorzugt Gegenstand der Erfindung.

### Hintergrund und Stand der Technik

Modulierbare Infrarot-Emitter (IR-Emitter) sind für eine Vielzahl von Anwendungen der Spektroskopie relevant. Insbesondere die Spektroskope von Gasen wird häufig mit Hilfe von Infrarotstrahlung durchgeführt. Elektromagnetische Strahlung im Infrarotbereich löst bei bestimmten Frequenzen bzw. Wellenlängen Vibrationen der beteiligten Gasmoleküle aus, die sich durch Absorptionslinien im Spektrum detektieren lassen.

Beispielsweise ist die Umweltsensorik stark auf optische- bzw. spektroskopische Systeme angewiesen, welche im mittleren Infrarot-Bereich - also bei Wellenlängen von 2 µm bis 10 µm - arbeiten, da sich hier die Infrarotbanden vieler bedeutender Stoffe wie z.B. Kohlenstoffdioxid oder Methan befinden.

Häufig kommt die photoakustische Spektroskopie zum Einsatz, bei der intensitätsmodulierte Infrarotstrahlung mit Frequenzen im Absorptionsspektrum eines in einem Gas zu detektierenden Moleküls eingesetzt wird. Ist dieses Molekül im Strahlengang vorhanden, findet eine modulierte Absorption statt, die zu Erwärmungs- und Abkühlungsprozessen führt, deren Zeitskalen die Modulationsfrequenz der Strahlung widerspiegeln. Die Erwärmungs- und Abkühlungsprozesse führen zu Expansionen und Kontraktion des Gases, wodurch Schallwellen mit der Modulationsfrequenz verursacht werden. Diese lassen sich durch Schalldetektoren (Mikrofone) oder Flusssensoren messen.

Die photoakustische Spektroskopie erlaubt die Detektion feinster Konzentrationen von Gasen und hat eine Vielzahl von Anwendungen. Beispielhaft ist die Detektion von CO₂, welche in der Forschung und der Klimatechnik eine Rolle spielt. Auch die Konzentration z. B. von Abgasen in der Luft kann so gemessen werden. Ebenso sind militärische Anwendungen relevant, bei denen kleinste Konzentrationen von Giftgas detektiert werden können.

Verschiedene Emitter werden als Strahlungsquelle für die genannten Anwendungen verwendet, mit unterschiedlichen Vor- und Nachteilen. Es können beispielsweise schmalbandige Laserquellen im Infrarotbereich verwendet werden. Diese erlauben die Verwendung hoher Strahlungsintensitäten und können mit Standardkomponenten hochfrequent moduliert werden, z.B. für die photoakustische Spektroskopie. Der Aufbau ist jedoch aufwändig und teuer. Insbesondere zur Detektion verschiedener Moleküle, muss eine entsprechende Anzahl von Lasern verwendet werden.

Auch ist es bekannt auf Basis optischer Nanostrukturen schmalbandige thermische Emitter bereitzustellen. Inoue et al. 2015 offenbart eine Reihe verschiedener Ansätze ([8]). Mit Verweis auf Landy et al. 2008 wird die Verwendung von Metamaterialien für selektive Infrarot Emitter diskutiert ([13]). Beispielsweise wird die Bereitstellung eines single-band und dual-band IR-Emitter mittels Metamaterialen offenbart. Hierdurch können kann eine wellenlängenspezifische Transmission mit hohen Emissionsgraden und Q-Faktoren gewährleistet werden. Eine hochfrequente Modulation der abgestrahlten Infrarotstrahlung ist im Gegensatz zu den vorbeschriebenen Laserquellen nicht möglich.

Thermische, breitbandige Emitter sind ebenso bekannt. Diese haben den Vorteil eines breiten Spektrums und oft geringer Kosten. Jedoch ist die Modulationsfrequenz dieser Emitter beschränkt, eine direkte Modulation durch Variation der Stromzufuhr ist aufgrund thermischer Zeitkonstanten langsam und verschlechtert die Lebensdauer des Bauteils erheblich. Eine langsame Modulation hat oft eine Messung mit einem schlechten Signal-zu-Rauschverhältnis zur aufgrund des Eigenrauschens der Detektionskomponenten zur Folge. Eine externe Modulation durch die Verwendung sich drehender Chopperräder ist schneller, jedoch ist der Aufbau aufwendig und nicht derart kompakt und robust, wie es für viele Anwendungen wünschenswert wäre. Auch sind die Modulationsbandbreiten beschränkt und eine Variation der Rotationsgeschwindigkeit des Choppers ist aufgrund von Trägheiten schwerfällig.

Gleichzeitig gibt es ein starkes Interesse an der Miniaturisierung dieser IR-Sensorelemente, um deren Integration in möglichst viele technische Anwendungen realisieren zu können.

Für die Miniaturisierung am besten geeignet sind Sensoren bzw. Spektrometer, deren Funktion auf dem photoakustischen Effekt beruht, da es sich um ein Verfahren der Absorptionsspektroskopie handelt und somit deutliche kleinere optische Weglängen für ein ausreichenden Signal-Rausch-Verhältnis genügen, im starken Gegensatz zu Verfahren der Transmissionsspektroskopie [7]. Gleichwohl erfordert dies aber auch die Verfügbarkeit miniaturisierter Infrarot-Emitter, welche einerseits eine möglichst hohe Intensität erreichen sollen - da diese zu einer direkten Erhöhung des Messsignals führt - , andererseits aber auch mit möglichst hohen Frequenzen moduliert werden müssen. Die Modulation des Emitters ist unabdingbar für das Auftreten des photoakustischen Effekts, da andernfalls kein akustisches Signal im zu untersuchenden Medium entsteht. Parallel hierzu bedeutet eine höhere Modulationsfrequenz des Emitters sowohl eine Steigerung der Funktionsgeschwindigkeit des photoakustischen Sensors, als auch eine Verbesserung des Signal-Rausch-Verhältnisses (SNR) [7].

Bisher kommen im Wesentlichen miniaturisierte thermische IR-Emitter zum Einsatz, sogenannte Microhotplates [5,6], welche bei Temperaturen von einigen hundert Grad betrieben werden und Modulationsfrequenzen von lediglich einigen Hz erreichen. Durch den Einsatz von plasmonischen Strukturen [8-10] gelingt es zwar die Spektren solcher thermischen Emitter positiv zu beeinflussen, das Grundproblem der hohen Temperaturen und geringen Modulationsfrequenzen bleibt jedoch bestehen und kann im Rahmen der vorhandenen Technologie nicht gelöst werden.

Für die Herstellung miniaturisierter, mechanisch-elektronischer Vorrichtungen wird heute auf vielen Anwendungsgebieten auf die Mikrosystemtechnik zurückgegriffen. Die so herstellbaren Mikrosysteme (engl. *microelectromechanical system,* kurz MEMS) sind sehr kompakt (Mikrometerbereich) bei gleichzeitig hervorragender Funktionalität und immer geringeren Herstellungskosten. Beispielsweise werden in der DE 10 2017 206 183 A1 schnelle und kompakte Kammantriebe als MEMS-Aktuatoren beschrieben.

Auch wird teilweise bereits eine Kombination von Mikro-/Nanoelektromechanischen-Systemen mit Metamaterialien vorgeschlagen, welche ein enormes Potential für die Entwicklung neuartiger, abstimmbarer Bauelemente birgt. Dabei reichen die möglichen Anwendungsgebiete von so unterschiedlichen Bereichen wie der Kommunikations- und THz- Technologie bis zur Medizindiagnostik [1-4].

Aus Liu et al. 2017 ist eine Modulation der spektralen Emission von Oberflächen bekannt, welche zur Infrarottarnung oder Freund-Feind-Erkennung eingesetzt werden kann ([19]). Mittels eines Metamaterials ist die detektierbare Emission über einen Bereich verändert, welcher einer Temperaturänderung von 20°C entspricht. In Kombination mit MEMS-Aktuatoren können hierdurch dynamische Infrarotmuster auf Oberflächen generiert werden, um die entsprechenden Objekte eine Infrarotkamera-Erkennung zu vermeiden oder eine Kennzeichnung zu ermöglichen. Eine Verwendung von MEMS-Technologie auf Basis von Metamaterialien zur Modulation eines heizbaren IR-Emitters, welcher für die Anwendung in der photoakustischen Spektroskopie geeignet ist, ist im Stand der Technik unbekannt.

### Aufgabe der Erfindung

Aufgabe der Erfindung ist es, einen modulierbaren Infrarot-Emitter sowie ein Verfahren zur Erzeugung modulierter Infrarotstrahlung bereitzustellen, welche die Nachteile des Standes nicht aufweisen. Insbesondere war es eine Aufgabe der Erfindung einen hochfrequent und variabel modulierbaren Infrarot-Emitter zur Verfügung zu stellen, welcher sich gleichzeitig durch einen einfachen, kostengünstigen kompakten Aufbau auszeichnet.

### Zusammenfassung der Erfindung

Die Aufgabe wird durch die Merkmale des unabhängigen Anspruchs 1 gelöst. Bevorzugte Ausführungsformen der Erfindung sind in den abhängigen Ansprüchen beschrieben.

Durch den Aktuator des modulierbaren Infrarot-Emitters kann auf besonders schnelle und einfache Weise eine Modulation der Intensität der emittierten Infrarotstrahlung erreicht werden. Im Gegensatz zu bekannten Intensitätsmodulationen bei Infrarot-Emittern durch Variation der Stromzufuhr ist die erfindungsgemäße Modulation nicht durch thermische Zeitkonstanten begrenzt. Vielmehr können beispielweise MEMS-Aktuatoren genutzt werden, um Modulationsfrequenzen von deutlich über 100 Hz bis hin 100 kHz zu erreichen. Derartige Modulationsfrequenzen sind insbesondere für die photoakustische Spektroskopie vorteilhaft. Der modulierbare Infrarot-Emitter eignet sich jedoch darüber hinaus für jeglichen Anwendungen, bei denen eine schnelle und zuverlässige Modulation einer Infrarot-Strahlung gefordert ist.

Wie im Folgenden im Detail erläutert, basiert die Modulation der Emission der Infrarotstrahlung auf der Ausnutzung der Eigenschaften des strukturierten Metamaterials des Deckelements. In der zweiten Position befindet sich das Deckelement aus dem strukturierten Metamaterial bevorzugt in einem lediglich durch die dielektrischen Zwischenschicht begrenzten, möglichst nahen Abstand mit einem möglichst großen Überlappungsgrad oberhalb des Grundelementes. In dieser zweiten (resonanten) Position kann Infrarotstrahlung an das Schichtsystem aus Deckelement, dielektrischer Zwischenschicht und Grundelement koppeln, sodass bei einer bevorzugten infraroten Resonanzwellenlänge ein besonders hoher Emissionsgrad auftritt bzw. eine Infrarotemission mit hoher Intensität erfolgt.

In der ersten Position erfolgt bevorzugt keine resonante Kopplung, sodass der IR-Emitter bei der Resonanzwellenlänge, einen deutlich verminderten Emissionsgrad aufweist bzw. eine Infrarotemission mit deutlich verminderter Intensität erfolgt. In Bezug auf die Resonanzwellenlänge kann durch einen mittels des Aktuators vermittelten Positionswechsel zwischen einem resonanten Zustand (zweite Position) und einem nicht-resonanten Zustand (erste Position) die Intensität der Emission der Infrarotstrahlung schnell und zuverlässig moduliert werden.

Hierzu kann beispielsweise der Aktuator so konfiguriert sein, dass dieser den vertikalen Abstand zwischen Deckelement und Grundelement von einer ersten, nicht-resonanten Position in eine zweite, resonante Position absenkt. Auch ist es möglich, dass der Aktuator das Grundelement und Deckelement horizontal gegeneinander verschiebt, wobei im Falle einer hinreichenden Überdeckung in einer zweiten Position eine resonante Emission erfolgt. Durch diese schnelle und zuverlässige Modulationsfähigkeit grenzt sich der IR-Emitter deutlich von bekannten Infrarot-Emittern des Standes der Technik ab.

Im Sinne der Erfindung bezeichnet ein modulierbarer Infrarot-Emitter eine Vorrichtung, die elektromagnetische Strahlung aussendet. Diese Strahlung hat bevorzugt einen Wellenlängenbereich im Infrarot (IR) Bereich, insbesondere zwischen ca. 700 Nanometer (nm) und 1 Millimeter (mm) Wellenlänge. Die dementsprechende Frequenz der emittierten Strahlung kann im Bereich zwischen etwa 300 Gigahertz (GHz) bis 400 Terrahertz (THz) liegen. Das Spektrum kann ebenso bevorzugt anhand der Wellenzahl m⁻¹ bzw. cm⁻¹ wiedergegeben werden, wie es im Bereich der Spektroskopie üblich ist. Ein Fachmann weiß, wie die Umrechnung in diese Einheiten vorgenommen wird.

Das Spektrum ist insbesondere so gewählt, dass es dem bevorzugten Anwendungsgebiet des Emitters, nämlich der Infrarotspektroskopie und insbesondere der photoakustischen Spektroskopie entspricht. Dabei ist insbesondere die Schwingungsanregung der zu spektroskopierenden und/oder zu detektierenden Gasmoleküle bevorzugt, welche je nach Gasmolekülen einem bevorzugten spektralen Bereich entsprechen. Beispielsweise ist für die Anregung von CO₂ Molekülen ein Spektralbereich von etwa 2,4 Mikrometern (µm) geeignet. Besonders bevorzugte Wellenlängenbereich der Infrarotstrahlung sind 700 nm bis 10 µm, bevorzugt 1 bis 10 µm, besonders bevorzugt 2 µm bis 10 µm.

Die Emission des IR-Emitters erfolgt bevorzugt als Strahl, welcher in einer bevorzugten Richtung in Form einer Gerade orientiert ist. Die Orientierung des Strahles wird dabei bevorzugt durch die Flächennormale zum Grundelement, der dielektrischen Zwischenschicht und dem Deckelement vorgeben, welche durch das Heizelement zur Infrarot-Strahlung angeregt werden. Der Begriff Strahl soll im weiteren Verlauf den vorzugsweise gebündelten Teil der Strahlung entlang der bevorzugten Strahlrichtung des Emitters beschreiben, welcher vom Emitter ausgesandt wird, wobei insbesondere die Bereiche der größten Intensität entlang dieser Richtung den Strahl definieren. Intensität ist bevorzugt definiert als Flächenleistungsdichte und hat bevorzugt die Einheit Watt pro Quadratmeter oder abgekürzt W/m².

Der Aufbau des modulierbaren Infrarot-Emitters legt eine bevorzugte Emissionsrichtung eines Infrarotstrahl entlang der Flächennormale des Schichtaufbaus auf dem Heizelement fest. Es können jedoch zusätzliche Komponenten wie z. B. Linsen im Emitter integriert oder extern angebracht sein, die für eine Bündelung bzw. Kollimation des Strahls sorgen. Ein Fachmann weiß, wie er durch das Design des IR-Emitters sowie durch Verwendung weiterer Komponenten das Emissionsprofil der Strahlungsquelle so formt, dass ein gewünschtes Strahlprofil sowie eine gewünschte Strahlrichtung resultieren. Dabei kann der modulierbare IR-Emitter bevorzugt ohne zusätzliche Linsen auskommen, oder als ein System umfassend Strahlungsquelle und mindestens einer Linse zur Kollimation des Strahls vorliegen.

Der Emitter ist modulierbar, das bedeutet, dass die Intensität der emittierten Strahlung, bevorzugt die Intensität des Strahls im zeitlichen Verlauf kontrollierbar geändert werden kann. Die Modulation soll bevorzugt eine zeitliche Änderung der Intensität als messbare Größe hervorrufen. Das bedeutet z. B., dass die Intensität im zeitlichen Verlauf zwischen der innerhalb des Messzeitraums gemessenen schwächsten Intensität und der innerhalb desselben Zeitraums gemessenen stärksten Intensität ein Unterschied besteht, der größer ist als die Sensibilität eines für das Strahlungsspektrum und die Anwendung typischerweise verwendeten Geräts zur Messung oder Bestimmung der Intensität. Bevorzugt ist der Unterschied deutlich größer als ein Faktor 2, mehr bevorzugt 4, 6 oder 8 zwischen der stärksten und der schwächsten einstellbaren Intensität. Besonders bevorzugt erfolgt die Modulation der Intensität des modulierten Strahles für eine oder mehrere vorbestimmte Resonanzwellenlängen. Ein modulierbarer Infrarot-Emitter hat eine Vielzahl von Anwendungen. Als relevante Anwendung ist insbesondere jede Form von Infrarotspektroskopie und insbesondere die photoakustische Spektroskopie zu nennen.

Zur Erzeugung der Infrarotstrahlung ist die Bereitstellung thermischer Energie in Form eines Heizelements vorgesehen. Besonders bevorzugt ist ein Mikro-Heizelement. Unter einem Mikro-Heizelement wird bevorzugt ein Heizelement mit Abmessungen der Größenordnung Mikrometer (µm) verstanden. Dabei umfasst das Heizelement eine erhitzbare Schicht aus einem leitfähigen Material, welches bei Durchfluss eines elektrischen Stroms joulesche Wärme produziert. Die produzierte Wärme zeigt bevorzugt eine Abhängigkeit vom ohmschen Widerstand des Elements und vom Quadrat der Stromstärke bzw. vom Quadrat der angelegten Spannung und dem inversen ohmschen Widerstand, je nachdem, ob eine Strom- oder eine Spannungsquelle verwendet wird.

In einem Gleichgewichtszustand ist die produzierte Wärme gleich zu den Wärmeverlusten durch Wärmeleitung, Konvektion und Wärmestrahlung (synonym: thermische Strahlung, Infrarotstrahlung), welche an den äußeren Grenzflächen der stromdurchflossenen erhitzbaren Schicht abgegeben wird. Wie dem Fachmann bekannt ist, verursacht die produzierte Wärme unter anderem thermische Strahlung, insbesondere durch thermische Bewegung von Teilchen, welche z. B. eine Beschleunigung von Ladungsträgern und/oder oszillierende Dipolmomente zur Folge hat. Somit kann durch eine stromdurchflossene erhitzbare Schicht gezielt Infrarotstrahlung erzeugt werden. Die erhitzbare Schicht ist bevorzugt aus Metall, beispielsweise aus Wolfram oder aus Platin. Durch Anlegen einer geeigneten Spannung und den daraus resultierenden Stromfluss wird joulesche Wärme und somit letztendlich Infrarotstrahlung erzeugt.

Das Strahlungsspektrum eines erhitzen Körpers lässt sich dabei bevorzugt angenähert durch das Plancksche Strahlungsgesetz beschreiben, wobei dem Fachmann die Unterschiede einer realen erhitzbaren Schicht zu einem schwarzen Körper bekannt sind, beispielsweise der Emissionsgrad oder die reale Abweichung von einem thermischen Gleichgewicht des Körpers. Trotz dieser Abweichungen wird das erzeugte Spektrum und dessen Intensität im Wesentlichen von der Temperatur und der abstrahlenden Fläche gemäß dem Planckschen Strahlungsgesetz beschrieben.

Somit kann ein Fachmann durch gezieltes Design des Mikro-Heizelements ein bevorzugtes Spektrum mit einer bevorzugten Intensitätsverteilung erzielen. Hierzu sind neben dem Material und der geometrischen Ausgestaltung des Heizelements bevorzugt die zur Verfügung gestellte elektrische Energie, sowie die Größe der Wärmeverluste des Heizelements neben der Wärmestrahlung maßgeblich. Die Größe dieser Wärmeverluste wird beispielsweise bestimmt durch die Wärmeleitfähigkeit zwischen dem Heizelement und den angrenzenden Materialien und/oder Fluiden sowie deren Wärmekapazität und der Größe der Grenzfläche(n).

Das Mikro-Heizelement ist bevorzugt zumindest teilweise freistehend und erlaubt z. B. innerhalb des IR-Emitters thermische Dehnungen aufgrund von starken Temperaturänderungen sowie Translationsbewegungen. Teilweise freistehend bedeutet, dass es an den Grenzflächen zumindest teilweise nicht kraft- und/oder formschlüssig mit anderen Elementen des Emitters verbunden ist und daher einen Freiheitsgrad der Bewegung in eine im Wesentlichen zur Grenzfläche senkrechten Richtung aufweist.

Der erfindungsgemäße Infrarot-Emitter ist dadurch gekennzeichnet, dass auf dem Heizelement ein flächiges Grundelement, eine dielektrischer Zwischenschicht und flächiges Deckelement aus einem Metamaterial vorliegen.

Flächig meint bevorzugt eine Dimensionierung, wobei die Dicke des Grundelementes oder Deckelementes deutlich geringer ist als dessen Länge und/oder Breite. Beispielsweise kann der Begriff flächig eine Dicke von weniger als 1500 nm oder bevorzugt weniger als 500 nm meinen, die Elemente eine Länge oder Breite im Bereich hunderter Mikrometer oder mehrere Millimeter aufweisen, sodass ein Verhältnis der Dicke zur Länge bzw. Breite des Grundelementes oder Deckelementes von mehr als 1:10, mehr als 1:50 oder mehr als 1:100 bevorzugt gegeben ist.

Die Form des Grundelementes, der dielektrischer Zwischenschicht oder des Deckelementes kann beispielsweise rechteckig, quadratisch oder kreisförmig sein. Bevorzugt weisen Grundelement, dielektrische Zwischenschicht oder Deckelement jedoch im Wesentlichen gleiche Formen und im Wesentlichen gleiche Flächen auf.

Wie im Folgenden im Detail erläutert, beeinflusst der durch den Aktuator bewegbare Schichtaufbau aus Grundelement, dielektrischer Zwischenschicht und Deckelement auf dem Heizelement die Intensität der emittierten Infrarotstrahlung maßgeblich.

Während es in einer zweiten Position zu einer resonanten Abstrahlung einer Infrarotstrahlung kommt (der Emissionsgrad in Bezug auf eine Resonanzwellenlänge ist besonders hoch), ist eine erste relative Positionierung von Grundelement und Deckelement so gewählt, dass es zu keiner resonanten IR-Abstrahlung oder nur zur einer deutlich verminderten IR-Abstrahlung kommt.

Die Modulation des Emissionsgrades durch den Aktuator basiert somit auf einer elektromagnetischen Resonanz für die Emission der Infrarotstrahlung, welcher in der zweiten relativen Position von Deckelement und Grundelement im Gegensatz zur ersten Position auftritt. Zu diesem Zweck ist es bevorzugt, dass in der zweiten Position das Deckelement aus dem strukturierten Metamaterial sich in einem lediglich durch die dielektrischen Zwischenschicht begrenzten, möglichst nahen Abstand mit einem möglichst großen Überlappungsgrad über dem Grundelement befindet.

In der zweiten Position ist der IR-Emitter bevorzugt durch einen Aufbau gekennzeichnet, in welchem das Grundelement aus einem leitfähigen Material, im Wesentlichen nur durch die dielektrische Zwischenschicht von einem darüber liegenden Deckelement aus einem strukturierten Metamaterial getrennt ist und wobei das Deckelement die Fläche des Grundelementes zu einem bevorzugt maximalen Anteil überdeckt.

Bei geeigneter Wahl des Metamaterials bildet ein derartiger Schichtaufbau aus Grundelement, dielektrischer Zwischenschicht und Deckelement bevorzugt einen *metamaterial perfect absorber* oder zumindest einen Absorber, welcher einem *metamaterial perfect absorber sehr* nahe kommt. Ein *metamaterial perfect absorber* ist dadurch gekennzeichnet, dass eine einfallende elektromagnetische Strahlung nahezu vollständig absorbiert wird, d.h. in Bezug auf mindestens eine Resonanzwellenlänge eine Absorption von nahezu 1 aufweist.

Aufgrund des Kirchhoffschen Strahlungsgesetz fungiert ein derartiger Schichtaufbau vorteilhafterweise im Kontext des erfindungsgemäßen Aufbaus des IR-Emitters auch als nahezu perfekter IR-Emitter. Das Kirchoffeschen Strahlungsgesetz besagt, dass für alle Körper im thermischen Gleichgewicht bei gegebener Temperatur das Verhältnis zwischen Emission und Absorption A für Strahlung derselben Wellenlänge konstant und vom Betrag gleich der spezifischen Ausstrahlung des schwarzen Körpers bei dieser Temperatur. Anders ausgedrückt wird ein Material mit einem für eine bestimmte Wellenlänge hohen Absorptionsgrad für die Wellenlänge auch einen hohen Emissionsgrad aufweisen.

Das Deckelement aus Metamaterial, die dielektrische Schicht und das Grundelement können derart gewählt werden, dass in der zweiten Position eine resonante Absorption bei mindestens einer bevorzugten Infrarotwellenlänge auftritt. In dieser Position wird der IR-Emitter die durch das Heizelement bereitgestellte thermische Energie resonant mit mindestens jener Resonanzwellenlänge emittieren. Die erste Position kann vorteilhafterweise so gewählt werden, dass die Resonanz nicht auftritt und der IR-Emitter über ein breites Infrarotspektrum nur einen geringen Emissionsgrad aufweist.

In Bezug auf die Resonanzwellenlänge kann durch einen mittels des Aktuators vermittelten Positionswechsel zwischen einem resonanten Zustand (zweite Position) und einem nicht-resonanten Zustand (erste Position) der Emissionsgrad schnell und zuverlässig moduliert werden. Wie im Folgenden näher erläutert, ist es mit MEMS-Aktuatoren beispielsweise möglich Modulationsfrequenzen von mehreren kHz zu erreichen. Die Kombination eines bewegbaren Deckelementes aus einem strukturierten Metamaterial und einem durch ein Heizelement erhitzbaren Grundelement, erlaubt mithin die Bereitstellungen eines hochfrequenten IR-Emitters mit hoher Modulationstiefe.

Vorteilhafter ergibt sich hierdurch für Anwendungen in der photoakustischen Spektroskopie ein ausgezeichnetes Signal-Rausch-Verhältnis bei schnellen Geschwindigkeiten. Während bei bekannten modulierbaren IR-Emittern, die Geschwindigkeit durch die Modulationsfrequenz des IR-Emitters limitiert ist, ist mit dem beschriebenen hochfrequent modulierbaren IR-Emitter die physikalische Obergrenze bevorzugt lediglich durch die Relaxationszeiten der Elektronen im untersuchten Gas gegeben.

Die Wahl einer oder mehrere Resonanzwellenlängen kann durch geeignete Wahl eines strukturierten Metamaterials erfolgen.

Im Sinne der Erfindung werden die Begriffe "strukturiertes Metamaterial" oder "Metamaterial" bevorzugt synonym verwandt und bezeichnen eine künstlich hergestellte Struktur, deren Durchlässigkeit für elektrische und magnetische Felder, d.h. deren elektrische Permittivität (ε(w)) oder magnetischen Permabilität (µ(w)), frequenzabhängig und anwendungsorientiert eingestellt werden kann. Insbesondere können strukturierte Metamaterialien somit elektromagnetischen Eigenschaften aufweisen, welche bei natürlichen Materialien nicht anzufinden sind.

Beispielsweise wurde bereits im Jahr 1968 gezeigt, das Metamaterialien einen negativen Brechungsindex erreichen können [11].

Bevorzugt wird das strukturierte Metamaterial aus einem Array von periodischen Einheitszellen gebildet, deren Dimensionierung geringer ist, als die Wellenlänge der elektromagnetischen Strahlung, welche durch das Metamaterial in gewünschter Weise beeinflusst werden soll. Unter einer "Einheitszelle" oder "Elementarzelle" wird bevorzugt analog zu bekannten Elementarzellen eines Kristalls eine kleinste geometrische Einheit des strukturierten Metamaterials verstanden, welche sich in der flächigen Ebene des Metamaterials periodisch wiederholt. Bevorzugt bildet eine zweidimensionale, periodische Anordnung aller Einheitszelle die Fläche des strukturierten Metamaterials.

Als Materialien für das Metamaterial eignen sich insbesondere hochleitfähige Metalle, wie beispielsweise Gold, Silber oder Kupfer, welche beispielsweise als *split-ring resonators* angeordnet werden können [12].

Derartige Resonatoren eignen sich insbesondere auch zur Ausbildung eines *metamaterial perfect absorbers.* Beispielsweise kann wie von *Landy et al.* gezeigt, ein *metamaterial perfect absorber* von zwei metallischen Schichten gebildet werden, die von einer dielektrischen Zwischenschicht getrennt werden. Die obere Metallschicht kann mehrere sogenannte *electric ring resonators* (EER) aufweisen, während die untere Metallschicht durch einen homogenen Streifen gebildet wird [13]. Bei einem solchen Aufbau kommt es zu einer elektrischen Kopplung mit der oberen Metallschicht und einer magnetischen Kopplung mit antiparalleler Ströme in beiden Schichten und einer Lorentz-ähnlichen magnetischen Antwort. In dem ursprünglichen Aufbau wird eine Resonanz im Mikrowellenbereich realisiert. Durch eine entsprechende Dimensionierung der Einheitszelle kann auch eine Resonanz der Absorptionen im Infrarotbereich erreicht werden [14].

Der Fachmann kennt verschiedenen Vorzugsformen von Metamaterialien und Aufbauten für *metamaterial perfect absorbers,* welche zur Ausbildung einer resonanten Absorption bei einer gewünschten Wellenlänge eignen [15]. Beispielsweise kann auch ein hexagonaler Array von kreisförmigen Strukturen als ein Metamaterial oberhalb einer homogenen Grundplatte verwandt werden [16]. Auch rechteckige oder kreuzförmige Resonatoren können für einen *metamaterial perfect absorber* im Sinne der Erfindung bevorzugt verwandt werden.

Wie im Folgenden im Detail erläutert, wird das strukturierte Metamaterial, die dielektrische Zwischenschicht und das Grundelement bevorzugt derart ausgestaltet, dass eine resonante Emission bei einer resonanten Wellenlänge im Infrarotbereich, bevorzugt in einem Wellenlängenbereich von 1 µm bis 10 µm, bevorzugt 2 µm bis 10 µm auftritt.

Neben den beschriebenen bevorzugten Ausführungsformen für ein Deckelement aus einem Metamaterial, Grundelement und einer dielektrische Zwischenschicht kann der Fachmann auf Basis numerischer Simulation auch weitere geeignete Strukturierungen, Dimensionierungen und/oder Materialien finden. Die elektromagnetische Theorie sowie Gleichungssysteme zur numerischen Lösung sind ihm bekannt [15].

Das Schichtsystem aus Grundelement, dielektrischer Zwischenschicht und Deckelement wird hierbei so bevorzugt ausgewählt, dass in der zweiten (resonanten) Position für eine gewünschte Resonanzwellenlänge ein besonders hoher Emissionsgrad erreicht wird.

Für einen *metamaterial perfect absorber* kann die Energiebilanz, wie folgt zusammengefasst werden: T (ω) + R(ω) + A (ω) = 1, wobei T die Transmission ist, R die Reflektion und A die Absorption. Die Parameter des Deckelementes, des Grundelementes und der dielektrischen Zwischenschicht, werden bevorzugt so gewählt, dass in der zweiten Position A (ω) maximiert, wodurch gemäß des Kirchhoffeschen Gesetzes auch eine resonante Emission bei der Frequenz ω auftritt und der Emissionsgrad maximiert wird.

Der Emissionsgrad eines Körpers gibt dabei bevorzugt an, wie viel Strahlung ein Körper im Vergleich zu einem idealen Wärmestrahler, einem schwarzen Körper, abgibt. In der zweiten Position bei Vorliegen eines nahezu perfekten Absorbers auf dem Heizelement kann ein besonders hoher Emissionsgrad von theoretisch bis zu 1 erreicht werden.

In einem Beispiel ist der modulierbare Infrarot-Emitter dadurch gekennzeichnet, dass die Einheitszelle des strukturierten Metamaterials einen Resonator umfasst, welcher durch Streben des leitfähigen Materials geformt wird, wobei der Resonator bevorzugt die Form eines *split ring resonators* (SRR), *electric ring resonator* (ERR), eines Kreuzes, eines Quadrates, eines Kreises, eines Hexagons und/oder Kombinationen dieser Formen aufweist.

Ein Resonator bezeichnet bevorzugt eine Struktur aus Streben eines leitfähigen Materials, welche an ein elektrisches Feld koppeln kann. Insbesondere die vorgenannten Formen von Resonatoren in Form von *split ring resonators* (SRR), *electric ring resonator* (ERR), Kreuzen, Quadraten, Kreises und/oder Hexagonen eignen sich besonders zur Ankopplung an das elektrische Feld einer Infrarotstrahlung. Es können jedoch auch weitere Formen von elektrischen Resonatoren bevorzugt sein, um zur gewünschten resonanten Emission zu gelangen. Derartige Formen sind dem Fachmann beispielsweise aus der US 2013/0314765 A1 bekannt, wobei der Fachmann weiß, wie die Dimensionierung anzupassen ist, um eine Resonanz bei einer gewünschten Wellenlänge, bevorzugt im Bereich von 1 µm bis 10 µm, bevorzugt 2 µm bis 10 µm zu erreichen.

In einer bevorzugten Ausführungsform bilden die Einheitszellen ein zwei dimensionales periodisches Gitter, wobei der Gitterwinkel zwischen 60° und 120°, bevorzugt 90° beträgt und die beiden Gitterkonstanten zwischen 5% und 40 %, bevorzugt 10 % und 25 % einer Resonanzwellenlänge betragen, wobei die Resonanzwellenlänge ausgewählt ist aus einem Bereich zwischen 1 µm bis 10 µm, bevorzugt 2 µm bis 10 µm.

Die bevorzugte Dimensionierung der Einheitszellen orientiert sich somit an der gewünschten Resonanzwellenlänge, bei welcher ein hoher Emissionsgrad vorliegt und eine Emission mit besonders hoher Intensität erfolgt.

Für die besonders bevorzugten Resonanzwellenlängen in einem Bereich zwischen 2 µm bis 10 µm, liegen bevorzugte Gitterkonstanten in einem Bereich zwischen 100 nm und 4 µm, besonders bevorzugt 200 nm und 2,5 µm. Auch Zwischenbereiche aus den vorgenannten Bereichen können bevorzugt seien wie beispielsweise 100 nm bis 200 nm, 200 nm bis 300 nm, 300 nm bis 400 nm, 400 nm bis 500 nm, 600 nm bis 700 nm, 700 nm bis 800 nm, 800 nm bis 900 nm, 900 nm bis 1000 nm, 1000 bis 1100 nm, 1100 bis 1200 nm, 1300 bis 1400 nm, 1400 nm bis 1500 nm, 1600 nm bis 1700 nm, 1700 nm bis 1800 nm, 1800 nm bis 1900 nm, 1900 nm bis 2000 nm, 2000 nm bis 2100 nm, 2100 nm bis 2200 nm, 2200 nm bis 2300 nm, 2300 nm bis 2400 nm, 2400 bis 2500 nm, 2600 bis 2700 nm, 2700 bis 2800 nm, 2800 nm bis 2900 nm, 2900 nm bis 3000 nm, 3000 bis 3100 nm, 3100 bis 3200 nm, 3300 bis 3400 nm, 3400 nm bis 3500 nm, 3600 nm bis 3700 nm, 3700 nm bis 3800 nm, 3800 nm bis 3900 nm, 3900 nm bis 4000 nm. Ein Fachmann erkennt, dass die vorgenannten Bereichsgrenzen auch kombiniert werden können, um weitere bevorzugte Bereich zu erhalten, wie beispielsweise 200 nm bis 800 nm, 1000 nm bis 1500 µm oder auch 1400 nm bis 2500 nm.

Unter den Begriffen Gitterwinkel und Gitterkonstanten werden bevorzugt die üblichen Gitterparameter zur Beschreibung eines (zweidimensionalen) Gitters verstanden. Bekanntermaßen kann ein zweidimensionales Gitter durch periodisches Verschieben einer Einheitszelle, um jeweils denselben Abstand in zwei bestimmte Raumrichtungen, d.h. entlang zweier Gittervektoren, erzeugt werden. Der Gitterwinkel entspricht bevorzugt dem Winkel zwischen den beiden Gittervektoren, während die die Gitterkonstanten die Länge Einheitszellen in den beiden Dimensionen bzw. den Abstand zwei Einheitszellen entlang der Verschiebungsrichtung kennzeichnen.

Die genannten Gitterwinkel und Gitterkonstanten erweisen sich als besonders vorteilhaft zur Ausbildung einer elektromagnetischen resonanten Emission der IR-Strahlung bei Wellenlängen im Bereich von 1 µm bis 10 µm, bevorzugt 2 µm bis 10 µm. Die Ausführungsform ist mithin besonders für Anwendung in der Infrarotspektroskopie, insbesondere für die photoakustische Spektroskopie geeignet.

Die Wahl der Gitterkonstanten hängt bevorzugt von der gewünschten Resonanzwellenlänge ab. Für bevorzugte Resonanzwellenlängen in einem Bereich zwischen 2 µm und 3 µm, kann beispielsweise in bevorzugt sein Gitterkonstanten aus einem Bereich zwischen 100 nm und 1,2 µm, besonders bevorzugt 200 nm und 750 nm auszuwählen.

Besonders bevorzugt sind beide Gitterkonstanten im Wesentlichen gleich, sodass ein besonders hoher Emissionsgrad bei einer bestimmten Resonanzwellenlänge erzielt werden kann.

Es kann aber auch bevorzugt sein, in den unterschiedlichen Raumrichtungen unterschiedliche Gitterkonstanten zu wählen. Hierdurch ist es vorteilhafterweise möglich einen modulierbaren IR-Emitter bereitzustellen, der in der zweiten (resonanten) Position eine IR-Strahlung mit einem hohem Emissionsgrad in einem Bereich von mindestens zwei Resonanzwellenlängen aufweist. Hierdurch kann der modulierbare IR-Emitter vorteilhafterweise gleichzeitig eine IR-Strahlung zur photoakustischen Spektroskopie zweier oder mehr Gasen bereitstellen. Eine erste Resonanzwellenlänge kann beispielsweise bei ungefähr 2,4 µm liegen, um CO₂ zu detektieren, während eine zweite Resonanzwellenlänge ungefähr 3 µm betragen kann, um beispielsweise Methan zu detektieren. Insbesondere für spektroskopische Anwendung, wie die photoakustische Spektroskopie zur Detektion von Gasmolekülen, eröffnet die erfindungsgemäße Verwendung eines Metamaterials in einem IR-Emitter vielfältige Anwendungsmöglichkeiten mit hoher wirtschaftlicher Relevanz.

In einer bevorzugten Ausführungsform ist das Deckelement aus einem Metall, besonders bevorzugt aus Gold, Silber, Aluminium, Wolfram, Molbidän, Titan und/ oder Kupfer gefertigt ist.

In einer weiteren bevorzugten Ausführungsform ist das Grundelement aus einem Metall, besonders bevorzugt aus Gold, Silber, Aluminium, Wolfram, Molbidän, Titan und/ oder Kupfer gefertigt ist.

Besonders bevorzugt sind das Deckelement und das Grundelement aus einem Material gefertigt, welches eine im Wesentlichen gleiche elektrische Leitfähigkeit aufweist.

Begriffe wie im Wesentlichen, ungefähr, etwa, ca. etc. beschreiben bevorzugt einen Toleranzbereich von weniger als ± 20%, bevorzugt weniger als ± 10 %, noch stärker bevorzugt weniger als ± 5% und insbesondere weniger als ± 1%. Angaben von im Wesentlichen, ungefähr, etwa, ca. etc. offenbaren und umfassen stets auch den exakten genannten Wert.

In einer weiteren bevorzugten Ausführungsform sind das Grundelement und das Deckelement aus demselben Material gefertigt.

In einer weiteren bevorzugten Ausführungsform ist die dielektrische Zwischenschicht aus einem Material gefertigt ist ausgewählt aus einer Gruppe umfassend umfassend Aluminiumnitrid, Siliziumnitrid, Aluminiumoxid, Siliziumoxid, Titandioxid (TiO₂) und/oder Tantaloxid (Ta₂O₅).

Für die vorgenannte Wahl der Materialien für das Grundelement, die dielektrische Zwischenschicht und das Deckelement in Form eines strukturierten Metamaterials erfolgt eine besonders gute Abstimmung, bevorzugt eine besonders gute magnetische Kopplung, sodass hohe elektromagnetische Resonanzen und IR-Emissionen in der zweiten Position erreicht werden. Zudem zeichnen sich die Materialien durch hohe Beständigkeit gegenüber Korrosion, Oxidation oder mechanische Einflüssen aus, sodass ein langlebiger robuster IR-Emitter gewährleistet wird.

In einer bevorzugten Ausführungsform ist das Grundelement eine kontinuierliche leitfähige Schicht. Hierdurch wird die verlustbehafte Reflektionen vermindert und eine besonders gute Impedanzanpassung zwischen Deckelement und Grundelement erreicht.

In einer weiteren bevorzugten Ausführungsform werden das Grundelement, die dielektrische Zwischenschicht, sowie das Deckelement aus CMOS kompatiblen Materialien hergestellt. Hierdurch kann der modulierbare IR-Emitter mittels CMOS-Verschaltung auf einem einzigen Chip gefertigt werden. Mittels der möglichen Integration der CMOS-Technologie können somit stark miniaturisierte und leistungsfähige modulierbare IR-Emitter bereitgestellt werden. Zudem wird durch die CMOS-Technologie eine kostengünstige Serienfertigung ermöglicht, welche ein kommerziell attraktives Produkt sichert. Bevorzugte Materialien und Fertigungsschritte zur Verwendung der CMOS-Technologie zur Herstellung von MEMS-Geräten sind dem Fachmann beispielsweise aus Qu et al. bekannt [17].

In einer weiteren bevorzugten Ausführungsform weist das Deckelement eine Schichtdicke zwischen 100 nm und 1500 nm auf. Bevorzugte Schichtdicken liegen in dem vorgenannten Bereich, wobei auch Zwischenbereiche bevorzugt seien können wie beispielsweise 100 nm bis 200 nm, 200 nm - 300 nm, 300 nm - 400 nm, 400 nm bis 500 nm, 600 nm bis 700 nm, 700 nm bis 800 nm, 800 nm bis 900 nm, 900 nm bis 1000 nm, 1000 bis 1100 nm, 1100 bis 1200 nm, 1300 bis 1400 nm oder auch 1400 nm bis 1500 nm. Ein Fachmann erkennt, dass die vorgenannten Bereichsgrenzen auch kombiniert werden können, um weitere bevorzugte Bereiche zu erhalten, wie beispielsweise 100 nm bis 500 nm, 400 nm bis 1000 nm oder auch 800 nm bis 1500 nm.

In einer weiteren bevorzugten Ausführungsform weist die dielektrische Zwischenschicht eine Schichtdicke zwischen 100 nm und 1500 nm auf. Bevorzugte Schichtdicken liegen in dem vorgenannten Bereich, wobei auch Zwischenbereiche bevorzugt seien können wie beispielsweise 100 nm bis 200 nm, 200 nm - 300 nm, 300 nm - 400 nm, 400 nm bis 500 nm, 600 nm bis 700 nm, 700 nm bis 800 nm, 800 nm bis 900 nm, 900 nm bis 1000 nm, 1000 bis 1100 nm, 1100 bis 1200 nm, 1300 bis 1400 nm oder auch 1400 nm bis 1500 nm.. Ein Fachmann erkennt, dass die vorgenannten Bereichsgrenzen auch kombiniert werden können, um weitere bevorzugte Bereiche zu erhalten, wie beispielsweise 200 nm bis 600 nm, 300 nm bis 1000 nm oder auch 700 nm bis 1500 nm.

In einer weiteren bevorzugten Ausführungsform weist das Grundelement eine Schichtdicke zwischen 100 nm und 1500 nm auf. Bevorzugte Schichtdicken liegen in dem vorgenannten Bereich, wobei auch Zwischenbereiche bevorzugt seien können wie beispielsweise 100 nm bis 200 nm, 200 nm - 300 nm, 300 nm - 400 nm, 400 nm bis 500 nm, 600 nm bis 700 nm, 700 nm bis 800 nm, 800 nm bis 900 nm, 900 nm bis 1000 nm, 1000 bis 1100 nm, 1100 bis 1200 nm, 1300 bis 1400 nm oder auch 1400 nm bis 1500 nm. Ein Fachmann erkennt, dass die vorgenannten Bereichsgrenzen auch kombiniert werden können, um weitere bevorzugte Bereiche zu erhalten, wie beispielsweise 100 nm bis 400 nm, 400 nm bis 1200 nm oder auch 700 nm bis 1400 nm.

Die vorgenannten Schichtdicken eignen sich besonders um in der resonanten zweiten Position einen nahezu *perfect metamaterial absorber* bereitzustellen, welcher bei Erhitzung mittels des Heizelementes einen nahezu perfekten IR-Emitter bildet.

Die vorgenannten bevorzugten Ausführungsformen der Strukturierung des Metamaterials, sowie die Schichtdicken und Wahl der Materialien eignen sich besonders gut dafür in einer (zweiten) resonanten Position einen hohen Emissionsgrad und eine Abstrahlung mit hoher Intensität zu gewährleisten. Während theoretisch mittels eines nahezu *perfect metamaterial absorber* Emissionsgrade von bis zu 1 möglich sind, führen Fertigungstoleranzen oder Energieverluste in der Praxis zu geringeren maximalen Emissionsgraden in der resonanten Position. Vorteilhafterweise kann auch bei Emissionsgrade von deutlich weniger als 1 in der (zweiten) resonanten Position eine hohe Modulationstiefe erreicht werden. Die Modulationstiefe des modulierbaren IR-Emitters wird bevorzugt durch die Differenz des Emissionsgrad in einer zweiten im Vergleich zur ersten Position beeinflusst. D.h. für die mögliche Modulationstiefe des IR-Emitters ist nicht nur der in der zweiten (resonanten) Position maximal mögliche Emissionsgrad maßgeblich, sondern zudem der in der ersten (nicht-resonanten) Position bevorzugt geringere Emissionsgrad.

Die Erfinder haben erkannt, dass zur Einstellung des Emissionsgrades in einer ersten (resonanten) Position und in einer zweiten (nicht-resonanten) Position der vertikale Abstand zwischen Grundelement und Deckelement sowie deren Grad der Überlappung sich eignen.

In einer bevorzugten Ausführungsform ist der Aktuator daher konfiguriert für eine vertikale Translationsbewegung des Deckelementes und/oder Grundelementes entlang der Emissionsrichtung des Infrarot-Emitters, welche den Abstand zwischen dem Deckelement und dem Grundelement ändert. Bevorzugt ist der Abstand in der zweiten Position kleiner als in der ersten Position.

Bevorzugt liegen Deckelement und Grundelement in zwei parallelen Ebenen übereinander angeordnet vor. In einer ersten Position hält der Aktuator das Deckelement und/oder Grundelement bevorzugt einen hinreichend vertikalen Abstand, welche eine elektromagnetische Resonanz und die Ausbildung eines *metamaterial perfect absorbers* verhindert. Erst durch eine bevorzugte Verringerung des Abstandes durch eine vertikale Translationsbewegung, also beispielsweise ein Absenken des Deckelementes auf die dielektrische Zwischenschicht kommt es in einer zweiten Position zu einem hohem Emissionsgrad und einer resonanten Abstrahlung.

In einer bevorzugten Ausführungsform weist in der ersten Position das Deckelement einen Abstand zur dielektrische Schicht von mindestens 500 nm, bevorzugt mindestens 1000 nm auf und in der zweiten Position einen Abstand zur dielektrische Schicht von höchstens 200 nm, bevorzugt höchstens 50 nm, besonders bevorzugt 0 nm aufweist.

Die vorgenannten Abstände haben sich als besonders vorteilhaft erwiesen, um in der ersten Position im Gegensatz zur zweiten Position eine deutlich verringerte Emission zu gewährleisten. Ein vertikaler Abstand des Deckelementes zur dielektrischen Schicht von 0 nm, bezeichnet bevorzugt eine Kontaktierung des Deckelementes mit der dielektrischen Schicht. Dem Fachmann ist bekannt, dass auch bei einer unmittelbaren Kontaktierung aufgrund einer Rauheit der beiden kontaktierenden Flächen ein tatsächlicher Abstand von mehr als 0 nm vorliegen kann. Derartige Abweichungen seien von der offenbarten Ausführungsform erfasst.

Die Erfinder haben zudem erkannt, dass neben einer Variation des Abstandes durch eine vertikale Translationsbewegung, auch eine relative horizontale Translation von Deckelement und Grundelement zur Variation des Emissionsgrades und mithin der Intensität der Infrarotstrahlung genutzt werden kann.

In einer bevorzugten Ausführungsform ist der Aktuator konfiguriert für eine horizontale Translationsbewegung des Deckelementes und/oder des Grundelementes orthogonal zur Emissionsrichtung des Infrarot-Emitters, welche den Grad der Überdeckung zwischen dem Deckelement und dem Grundelement ändert. Bevorzugt liegt in der ersten Position ein geringer Grad der Überdeckung vor, als in der zweiten Position.

Unter dem Grad der Überdeckung wird bevorzugt das Verhältnis aus der Fläche des Grundelementes, über welche sich in Emissionsrichtung ein Abschnitt des Deckelementes befindet, zur Gesamtfläche des Grundelementes verstanden.

Ein Grad der Überdeckung von 0% bezeichnet somit einen Zustand, in welchem sich über dem Grundelement in Emissionsrichtung kein Abschnitt des Deckelementes aus strukturiertem Metamaterial befindet. Bei einem Grad der Überdeckung von 0% erfolgt keine resonante Abstrahlung der IR-Strahlung.

Ein Grad der Überdeckung von 100% bezeichnet hingegen eine relative Positionierung oder einen Zustand, in welchem sich über der gesamten Fläche des Grundelementes in Emissionsrichtung ein Abschnitt des Deckelementes aus strukturiertem Metamaterial befindet. Bei einem Grad der Überdeckung von 100% erfolgt eine resonante Abstrahlung der IR-Strahlung im Wesentlichen über die gesamte Fläche des Grundelementes. Bei einem Grad der Überdeckung zwischen 0 und 100% erfolgt eine resonante Abstrahlung bevorzugt lediglich über den überdeckten Anteil des Grundelementes.

Vorteilhafterweise kann somit auch durch eine horizontale Translationsbewegung und eine Variation des Grad der Überdeckung der Emissionsgrad bzw. die Intensität des emittierten IR-Strahles präzise moduliert werden.

In einer bevorzugten Ausführungsform der Erfindung weist in der ersten Position das Deckelement und Grundelement einen Grad der Überdeckung von weniger als 40%, bevorzugt weniger als 10%, besonders bevorzugt 0% auf, während in der zweiten Position das Deckelement und Grundelement einen Grad der Überdeckung von mehr als 40%, bevorzugt mehr als 80%, besonders bevorzugt 100 % aufweisen.

Die genannten Überdeckungsgrade lassen sich mechanisch sehr gut umsetzen und sichern hinreichende Modulationstiefen des IR-Emitters.

Bei einer horizontalen Translationsbewegung des Deckelementes gegenüber dem Grundelement zur Veränderungen des Grades der Überdeckung, ist der Aktuator bevorzugt so konfiguriert, dass der vertikale Abstand in der zweiten Position bevorzugt weniger als 200 nm, besonders bevorzugt weniger als 100 nm, mehr bevorzugt 0 nm beträgt. Zu diesem Zweck kann eine horizontale Translationsbewegung ohne zusätzliche vertikale Translationsbewegung erfolgen. Es kann aber auch bevorzugt sein, dass eine horizontale Translationsbewegung von einer vertikalen Translationsbewegung überlagert wird.

Im Sinne der Erfindung bezeichnet vertikal bevorzugt die Emissionsrichtung des IR-Emitters, welche durch die Flächennormale zum Deckelement und Grundelement gegeben ist. Wohingegen "horizontal" bevorzugt eine Richtung in einer Ebene meint, welche parallel zu den Ebenen liegt, in welchen das Deck- und Grundelement angeordnet vorliegen. Bezeichnungen wie übereinander, überdeckend etc. orientieren sich an der vertikalen Richtung, d.h. der Emissionsrichtung des IR-Emitter. Diese kann, muss aber nicht mit einer Gravitationsrichtung übereinstimmen.

Zur Gewährleistung der Relativbewegung von Grund- und Deckelement wird ein Aktuator verwandt. Bevorzugt setzt ein Aktuator insbesondere ein elektrisches Steuerungssignal in eine Bewegung um. Es kann sich dabei um einen MEMS-Aktuator handeln, welcher z. B ein elektrostatischer Aktuator ist.

Das Verhältnis zwischen der durch die Relativbewegung einstellbaren maximalen und minimalen Intensität der emittierten IR Strahlung kann als Extinktionsverhältnis bezeichnet werden. Es kann direkt aus dem Quotienten zwischen maximaler Intensität und minimaler Intensität bestimmt und bevorzugt direkt durch diesen Quotienten angegeben werden. Es kann aber auch bevorzugt sein, dass dieses Verhältnis in der logarithmischen Skala Dezibel (dB) ausgedrückt wird, wie es beispielsweise in der Nachrichtentechnik üblich ist. Der Fachmann versteht, dass das Extinktionsverhältnis je höher ist, desto höher die Differenz der in erster und zweiter Position einstellbaren Emissionsgrade.

Die bei den gewünschten Modulationstiefen erreichbaren maximalen Modulationsfrequenzen sollten bevorzugt mindestens 1 Kilo-Hertz (kHz), besonders bevorzugt mindestens 10 kHz, stärker bevorzugt mindestens 20 kHz, ganz besonders bevorzugt mindestens 30 kHz und insbesondere mindestens 100 kHz betragen. Besonders bevorzugt ist es, für eine Anwendung in der photoakustischen Spektroskopie Modulationsfrequenzen im Bereich des Hörschalls und/oder Ultraschalls zu erzielen. Die Modulationsbandbreite, über welche die gewünschte Modulationstiefe erreicht wird, betrifft bevorzugt den gesamten Frequenzbereich von 0 Hz bis zur maximalen Modulationsfrequenz.

Die gewünschte Modulation kann bevorzugt in einem entsprechenden zeitlichen Verlauf der emittierten Strahlungsintensität Ausdruck finden. Um die Umsetzbarkeit eines solchen gewünschten zeitlichen Intensitätsverlaufs zu ermitteln, sind insbesondere die Modulationstiefe und die Bandbreite über der diese Modulationstiefe im Wesentlichen erreichbar ist, von Bedeutung. Auch die Auflösung einer elektronischen Ansteuerung des IR-Emitters ist bevorzugt relevant für die Umsetzbarkeit. Dabei kann es bedeutsam sein, welche verschiedenen Intensitätsstufen zwischen minimaler und maximaler Intensität mit welcher Frequenz erreicht werden können.

Es ist bevorzugt, dass der IR-Emitter eine elektrische Ansteuerung aufweist, mit die durch den mindestens einen Aktuator hervorgerufene Relativbewegung von Grundelement und Deckelement reguliert wird. Eine solche kann z. B. über eine Steuerungseinrichtung realisiert werden. Durch eine Ansteuerung können die gewünschten Spektren, Intensitäten und Modulationen eingestellt werden.

Ansteuerung bedeutet bevorzugt, dass elektrische Steuersignale direkt an den Aktuator und das das Heizelement übermittelt werden, welche in den gewünschten Strahlungseigenschaften resultieren. Bei dem Heizelement kann so insbesondere eine bestimmte Temperatur und/oder ein bestimmter zeitlicher Temperaturverlauf eingestellt werden. Außerdem kann durch die vom Aktuator ausgelöste Relativbewegung (eventuell in Abstimmung mit einem Temperaturverlauf) ein bestimmtes Modulationssignal erzielt werden. Typischerweise handelt es sich um ein analoges Signal, welches von einer Steuereinrichtung erzeugt wird. Diese wiederum kann bevorzugt ein geeignetes, digitales, elektronisches Signal, beispielsweise durch einen Steuerungscomputer erhalten, welche dann vorteilhafterweise von der Steuereinrichtung in geeignete Ansteuerungssignale übersetzt wird.

Es ist besonders bevorzugt, dass weite Teile des IR-Emitters Abmessungen im Mikrometerbereich aufweisen, um einen miniaturisierten Aufbau zu ermöglichen.

In einer bevorzugten Ausführungsform ist das Heizelement ein Mikro-Heizelement.

In einer bevorzugten Ausführungsform ist der Aktuator ein MEMS-Aktuator, bevorzugt ausgewählt aus der Gruppe umfassend elektrostatischer Aktuator, piezoelektrischer Aktuator, elektromagnetischer Aktuator und/oder thermischer Aktuator.

Ein MEMS-Aktuator ist bevorzugt ein Aktuator, der mithilfe von üblichen Herstellungsmethoden der Mikrosystemtechnik hergestellt wird und des Weiteren vorteilhafterweise Abmessungen in der Größenordnung µm aufweist. Ein solcher Aktuator ist besonders kompakt, robust und wartungsarm und lässt sich einfach und kostengünstig herstellen. Insbesondere können große Teile des Emitters MEMS-Elemente, das heißt Elemente mit den bevorzugten, vorstehend genannten Eigenschaften sein und in einem Herstellungsschritt mit dem MEMS-Aktuator herstellbar sein. Es kann wünschenswerterweise in Teilen das gleiche Substrat zur Herstellung verwendet werden. Dies vereinfacht und verbilligt die Herstellung.

Die vorstehend genannten Aktuatoren sind besonders gut für eine große Anzahl schneller Translationsbewegungen geeignet und weisen insbesondere aufgrund des kompakten Aufbaus einen geringen Energiebedarf auf. Auch ist die Bandbreite der erzielbaren Translationsgeschwindigkeiten aufgrund des kompakten Aufbaus, geringer Trägheiten und der Linearbewegung sehr hoch.

In einer weiteren bevorzugten Ausführungsform des modulierbaren Infrarot-Emitters ist der MEMS-Aktuator ein elektrostatischer Aktuator in Form eines Kammantriebs basierend auf einer Variation der Kammüberdeckung und/oder des Kammabstands. MEMS-Kammantriebe sind aus dem Stand der Technik bekannt, z. B. aus der Patentanmeldung DE 10 2017 206 183 A1. Dabei kann je nach Ausführungsform die Kammüberdeckung und/oder der Kammabstand variiert werden. Es hat sich gezeigt, dass solche MEMS-Kammantriebe aufgrund ihrer Abmessungen und der erzeugbaren Bewegungen besonders geeignet sind für eine bevorzugte Translationsbewegung und einen kompakten IR-Emitter.

In einer weiteren bevorzugten Ausführungsform ist der Aktuator mit dem Deckelement gekoppelt und für eine Translationsbewegung des Deckelements gegenüber dem Grundelement konfiguriert. Eine Translationsbewegung bezeichnet insbesondere eine Verschiebung des Deckelementes oder Grundelementes. Diese soll bevorzugt vertikal entlang der Emissionsrichtung erfolgen. Gekoppelt bedeutet insbesondere, dass eine direkte mechanische Verbindung zwischen dem Deckelement und mindestens einem beweglichen Element des Aktuators besteht, so dass eine Bewegung des beweglichen Aktuatorelements eine Bewegung des Deckelements in die gewünschte Richtung auslöst.

Aktuator und Deckelement können dabei bevorzugt direkt miteinander verbunden sein. Es kann sogar bevorzugt sein, dass sowohl Aktuator als auch Deckelement dasselbe Substrat umfassen und/oder aus diesem gefertigt sind. Es kann dabei nicht nur eine mechanische, sondern ebenso eine thermische und/oder elektrische Kopplung zu dem Aktuator bestehen.

Es kann aber ebenso bevorzugt sein, dass der Aktuator mit dem Grundelement gekoppelt ist und für eine Translationsbewegung des Grundelements gegenüber dem Deckelement konfiguriert ist. Falls das Grundelement fest mit einer dielektrischen Schicht verbunden ist, kann der Aktuator auch indirekt über die dielektrische Zwischenschicht mit dem Grundelement verbunden werden.

Wie obig ausführlicher erläutert, kann für die Modulation der IR-Strahlung insbesondere eine Änderung des vertikalen Abstandes des Deck- und Grundelementes oder deren Grad der Überdeckung genutzt werden. In dieser Hinsicht eignen sich somit beide Elemente für eine Translationsbewegung.

Gleichwohl besonders bevorzugt ist es, dass das Heizelement, das Grundelement und die dielektrische Schicht mechanisch gekoppelt vorliegen und das Deckelement mittels des Aktuator bewegt wird. Hierdurch lässt sich der IR-Emitter zum einen besonders effizient herstellen und zum anderen erlaubt das geringe Gewicht des Deckelementes schnelle und präzise Translationsbewegungen.

In einer bevorzugten Ausführungsform liegt auf der dielektrischen Zwischenschicht ein Abstandsrahmen vor, dessen Höhe den vertikalen Abstand zwischen dielektrischer Zwischenschicht und Deckelement festlegt. Bevorzugt verläuft der Abstandsrahmen entlang der äußeren Umrandung der dielektrischen Zwischenschicht, wobei der innere Verlauf des Umfanges des Abstandsrahmens größer ist als der äußere Umfang des flächigen Deckelementes. Der mindestens eine Aktuator verbindet bevorzugt den Abstandsrahmen mit dem Deckelement, sodass beispielsweise in einer ersten Position, dass Deckelement auf Höhe des oberen Endes des Abstandsrahmens gehalten wird und der Aktuator für eine vertikale Absenkbewegung des Deckelements zur dielektrischen Zwischenschicht hin konfiguriert ist.

In dieser Ausführungsform ist das Heizelement, das Grundelement und die dielektrische Zwischenschicht bevorzugt ortsfest, wobei die Relativbewegung zwischen diesen und dem Deckelement durch eine Translationsbewegung des Deckelementes realisiert wird und die Bewegung von dem Aktuator ausgelöst wird. Eine Translationsbewegung bezeichnet in diesem Fall bevorzugt eine vertikale oder horizontale Verschiebung des Deckelements.

Aktuator und Abstandsrahmen können dabei bevorzugt direkt miteinander verbunden sein. Es kann sogar bevorzugt sein, dass sowohl der Abstandsrahmen, als auch der Aktuator dasselbe Substrat umfassen und/oder aus diesem gefertigt sind.

In einer bevorzugten Ausführungsform umfasst der Infrarot-Emitter mindestens vier MEMS-Aktuatoren, welche an äußeren Seiten des Deckelementes installiert vorliegen und konfiguriert sind die Relativbewegung des Deckelementes und des Grundelementes zwischen der ersten und zweiten Position simultan zu steuern. Durch die Verwendung von mindestens vier MEMS-Aktuatoren, welche an den äußeren Seiten des Deckelementes vorliegen kann eine besonders gleichmäßige und schnelle Translation des Deckelements gewährleistet werden.

Besonders bevorzugt können die mehreren MEMS-Aktuatoren an einem wie vorgehend beschriebenen Abstandsrahmen befestigt werden, welcher bevorzugt als eine Umrandung auf der dielektrischen Zwischenschicht aufgetragen ist und dessen Höhe den vertikalen Abstand von Deckelement zur dielektrischen Zwischenschicht in der ersten Position festlegt.

Die bevorzugte Anzahl der MEMS-Aktuatoren kann sich sich an der Form des Deckelementes orientieren. Im Falle eines flächigen, rechteckigen Deckelementes und einem entsprechend größer umrandenden rechteckigen Abstandsrahmen sind mindestens vier MEMS-Aktuatoren bevorzugt, wobei ein MEMS- Aktuator an jeder Seite installiert wird. Im Falle eines hexagonalen Deckelementes können bevorzugt entsprechend sechs MEMS-Aktuatoren verwandt werden, während im Falle einer dreieckigen Form auch drei MEMS-Aktuatoren bevorzugt sein können. Ganzzahlige Vielfache der jeweiligen Anzahl von MEMS-Aktuatoren sind ebenfalls bevorzugt, um beispielsweise pro äußerer Seite des Deckelementes jeweils 2, 3 oder mehr MEMS-Aktuatoren anzubringen.

In einer weiteren bevorzugten Ausführungsform weist der Infrarot-Emitter ein Gehäuse auf, in welchem das Heizelement, das Grundelement, die dielektrische Zwischenschicht, das Deckelement und der Aktuator installiert vorliegen.

Das Gehäuse kann sich dabei bevorzugt an den Abmessungen und Formen der installierten Elemente orientieren. Es kann ebenso bevorzugt sein, dass das Gehäuse bedeutend größer als die installierten Elemente ist, um die Handhabbarkeit des Emitters zu verbessern und eine robuste Vorrichtung zu schaffen. Beispielsweise können Heizelement, Aktuator, Grundelement, dielektrische Zwischenschicht und/oder Deckelement MEMS Elemente sein und/oder Abmessungen im (sub-) Mikrometerbereich haben, wobei das Gehäuse Abmessungen im Zentimeterbereich aufweist.

Bevorzugt ist, dass das Gehäuse eine durchgehende Außenfläche aufweist und nach innen geschlossen ist. Im Inneren des Gehäuses liegt insbesondere das Heizelement installiert vor. Dadurch kann dieses vor äußeren Einflüssen geschützt werden und eine Emission der IR Strahlung nach außen, außer in der bevorzugten Emissionsrichtung vermieden werden.

Es ist bevorzugt, dass das Mikro-Heizelement nicht thermisch isoliert von dem Gehäuse ist, sondern eine nichtstrahlende Wärmeabgabe vom Heizelement an das Gehäuse möglich ist, so dass Wärme vom Heizelement abfließen kann. So kann z. B. ein gewünschtes Gleichgewicht zwischen der erzeugten Wärme durch die stromdurchflossene, erhitzbare Schicht aus leitfähigen Material und der vom Heizelement an die Umgebung abgegebene Wärme hergestellt werden, die gewünschten Strahlungseigenschaften erzeugt werden und/oder die gewünschten Modulationseigenschaften erreicht werden.

Es kann beispielsweise bevorzugt sein, dass die Elemente Gehäuse und/oder Mikro-Heizelement aus demselben Material hergestellt werden und eine ausreichende Wärmeleitung zwischen direkt verbundenen Elementen besteht.

Das Gehäuse kann bevorzugt zur eigenen Wärmeableitung einen Kühlkörper aufweisen.

Es kann wünschenswert sein, bestimmte Elemente, wie das Deckelement, von dem Gehäuse, thermisch zu entkoppeln. Damit ist bevorzugt gemeint, dass durch Verwendung mindestens eines geeigneten Materials an der oder durch ein geeignetes Design eine Verbindungsstelle (beispielweise kleine Verbindungsfläche und/oder geeignete Dicke der Verbindung) das Deckelement sich nicht wesentlich aufheizt.

Ein geeignetes Material an der Verbindungsstelle umfasst bevorzugt die gesamte Verbindungsfläche. Geeignete Materialien beziehen sich dabei insbesondere auf die Wärmeleitfähigkeit der Materialien, ausgedrückt in Watt pro Meter und Kelvin (W/m·K).

Bevorzugte Wärmeleitfähigkeiten an der Verbindungsstelle betragen weniger als 10 W/m·K, besonders bevorzugt weniger als 1 W/m·K und insbesondere weniger als 0,1 W/m·K.

Beispielsweise kann eine Oxidschicht bevorzugt sein. Damit die direkte Übertragung von Wärme zwischen Mikro-Heizelement und Blendenstruktur minimiert wird, kann es bevorzugt sein, dass das Gehäuse konfiguriert ist für eine Erzeugung eines Vakuums im Inneren. Ein Vakuum bezeichnet bevorzugt einen Druck von weniger als 30 x 10³ Pascal (Pa), besonders bevorzugt von weniger als 100 Pa und insbesondere von 0,1 Pa und weniger. Konfiguriert bedeutet, dass das Gehäuse ausreichend druckdicht gestaltet ist.

Durch das resonante Abstrahlungsverhalten des IR-Emitters in der zweiten Position erfolgt bereits eine IR-Emission mit schmaleren Frequenzspektren um eine oder mehrere Resonanzwellenlänge. Je nach Verwendung des IR-Emitters, z. B. bei verschiedenen Spektroskopiemethoden, kann es jedoch wünschenswert sein noch schmalere Spektren zu nutzen. Zudem erfolgt insbesondere in der ersten Position, wenn auch mit geringer Intensität, eine Ausstrahlung über ein breiteres Frequenzspektrum.

Zur Selektion besonders schmalbandiger Spektren und zur Minimierung einer IR-Emission außerhalb der gewünschten schmalbandigen Spektren können bevorzugt Frequenzfilter verwendet werden. Bevorzugt werden diese in ein Gehäuse integriert.

Die verwendeten Filter können vorteilhafterweise verschiedene Filtereigenschaften aufweisen, z.B. können Bandpassfilter, Kurzpassfilter, Langpassfilter, Kerbfilter (engl. "Notchfilter") und jede beliebige Kombination dieser Filter, die zu den gewünschten spektralen Beeinflussungen führen, verwendet werden. Die Frequenzen bzw. Frequenzbereiche, in denen die Filter wirken, können beliebig je nach Anwendung gewählt werden. Als Filter kann beispielsweise ein Filterrad zum Einsatz kommen, auf dem Filter mit verschiedenen Filtereigenschaften eingebaut sind. So kann mechanisch, durch Drehung des Filterrads, der gewünschte Filter ausgewählt werden. Dabei kann das Filterrad bevorzugt durch einen elektrischen Antrieb gedreht werden.

Ebenso ist die Verwendung eines Fabry-Pérot Filters denkbar. Ein solcher Filter kann beispielsweise zur Selektion sehr schmaler Spektren verwendet werden. Bevorzugt ist das dem Filter zur Grunde liegende Fabry-Pérot Interferometer durchstimmbar, beispielsweise durch eine Abstimmbarkeit der Temperatur oder durch mechanische Anpassung. So können flexibel gewünschte Spektren selektiert werden.

Ebenso können vorzugsweise geeignete Dünnschichtfilter verwendet werden. Diese sind besonders einfach herzustellen und sind sehr kompakt. Insbesondere bei einer Herstellung des IR-Emitters in integrierter Bauweise in einem Herstellungsprozess kann die Herstellung eines solchen Dünnschichtfilters einfach in den Prozess integriert werden. Hierdurch werden die Kosten gesenkt.

Auch eine flexible Kombination von Dünnschichtfiltern bzw. ein durch z. B. Verfahren der Temperatur abstimmbarer Dünnschichtfilter ist vorteilhaft.

Es können auch Filter für andere Eigenschaften der IR-Strahlung, z. B der Polarisation, verwendet werden.

In einer bevorzugten Ausführungsform umfasst das Heizelement ein Substrat, auf welchem mindestens eine erhitzbare Schicht aus einem leitfähigen Material aufgebracht ist, an welchem Kontakte für eine Strom- und/oder Spannungsquelle vorliegen. Bevorzugt handelt es sich bei der erhitzbaren Schicht um eine separate Schicht, auf welche das Grundelement aufgebracht wird. Die erhitzbare Schicht kann aber auch durch das Grundelement gebildet werden.

Das Substrat bildet bevorzugt die Basis des Heizelements. Dabei kann das Substrat auch weitere Elemente des IR-Emitters, wie beispielsweise Aktuator, Grundelement, dielektrische Zwischenschicht, Deckelement und/oder Gehäuseelemente zumindest teilweise umfassen. Das Substrat kann vorteilhafterweise durch etablierte Prozessschritte, insbesondere aus der Halbleiter- und/oder Mikrosystemherstellung, geeignet geformt werden. Dann kann bevorzugt eine erhitzbare Schicht aus einem leitfähigen Material auf das Substrat aufgebracht bzw. in das Substrat integriert werden, z. B. durch Dotierung und/oder Beschichtung. Es ist bevorzugt, dass die erhitzbare Schicht zur Herstellung eines elektrischen Kontakts mit einer Quelle elektrischer Energie kontaktiert wird.

In einer weiteren bevorzugten Ausführungsform des modulierbaren Infrarot-Emitters ist das Substrat ausgewählt aus einer Gruppe umfassend Silizium, monokristallines Silizium,

Polysilizium, Siliziumdioxid, Siliziumcarbid, Siliziumgermanium, Siliziumnitrid, Nitrid, Germanium, Kohlenstoff, Galliumarsenid, Galliumnitrid und/oder Indiumphosphid. Diese Materialien sind in der Halbleiter- und/oder Mikrosystemherstellung besonders einfach und kostengünstig zu bearbeiten und eignen sich ebenfalls gut für eine Massenherstellung. Ebenso sind diese Materialien für ein Dotieren und/oder Beschichten besonders geeignet, um in bestimmten Bereichen die gewünschten elektrischen, thermischen und/oder Strahlungseigenschaften zu erzielen.

In einer weiteren bevorzugten Ausführungsform des modulierbaren Infrarot-Emitters ist das leitfähige Material zur Bildung der erhitzbaren Schicht ausgewählt aus der Gruppe umfassend Platin, Wolfram, (dotiertes) Zinnoxid, monokristallines Silizium, Polysilizium, Molybdän, Titan, Tantal, Titan-Wolfram Legierung, Metallsilizid, Aluminium, Graphit und/oder Kupfer. Diese Materialien weisen zum einen die gewünschten thermischen, elektrischen, mechanischen und/oder Strahlungseigenschaften auf und sind darüber hinaus besonders leicht und kostengünstig zu verarbeiten.

Ein Beispiel eines Herstellungsverfahrens für einen InfrarotEmitter wie vorstehend beschrieben umfasst folgende Schritte:
- Ätzen eines Substrats;
- Abscheiden eines leitfähigen Materials auf dem Substrat zur Bildung einer erhitzbaren Schicht sowie Kontaktierung der erhitzbaren Schicht;
- Abscheiden eines leitfähigen Materials zur Ausbildung des Grundelementes
- Abscheiden eines dielektrischen Materials zur Ausbildung einer dielektrischen Zwischenschicht
- Abscheiden eines leitfähigen Materials zur Ausbildung eines Deckelements und/oder Strukturierung des Deckelementes als Metamaterial mit periodisch angeordneten Einheitszellen, wobei bevorzugt

ein Ätzen und/oder eine Strukturierung ausgesucht ist aus der Gruppe umfassend Trockenätzen, nasschemisches Ätzen und/oder Plasmaätzen, insbesondere Reaktives Ionenätzen, Reaktives Ionentiefenätzen (Bosch-Prozess); und/oder
das Abscheiden ausgesucht ist aus der Gruppe umfassend physikalische Gasphasenabscheidung (PVD), insbesondere thermisches Verdampfen, Laserstrahlverdampfen, Lichtbogenverdampfen, Molekularstrahlepitaxie, Sputtern, chemische Gasphasenabscheidung (CVD) und/oder Atomlagenabscheidung (ALD).

Als Substrat kann z. B. eines der bevorzugten, vorstehend genannten Materialien verwendet werden. Beim Ätzen kann ein Rohling, beispielsweise ein Wafer, in die gewünschte Grundform eines Heizelements gebracht werden. In den nächsten Schritten kann das leitfähige Material für die erhitzbare Schicht, das leitfähige Material zur Ausbildung eines Grundelementes sowie eines leitfähigen Materials zur Ausbildung eines Deckelements abgeschieden werden.

Sollte eine weitere Strukturierung des leitfähigen Materials, insbesondere für das Deckelement gewünscht sein, kann diese z. B. durch weitere Ätzprozesse vorgenommen werden. Ebenso kann zusätzliches Material abgeschieden werden oder eine Dotierung durch übliche Verfahren vorgenommen werden.

Zur Kontaktierung der erhitzbaren Schicht kann zusätzlich geeignetes Material, wie z. B. Kupfer, Gold und/oder Platin auf dem leitfähigen Material durch gängige Prozesse abgeschieden werden. Hierfür können bevorzugt physikalische Gasphasenabscheidung (PVD), chemische Gasphasenabscheidung (CVD) oder elektrochemische Abscheidung zum Einsatz kommen.

Auf diese Weise können besonders feine Strukturen hergestellt werden, welche eine Dimensionierungen im Nano- oder Mikrometerbereich aufweisen. Ebenso haben sich diese Herstellungsschritte besonders bewährt und gehören zu Standardverfahrensschritten der Halbleiterprozessierung.

In einer weiteren bevorzugten Ausführungsform des Herstellungsverfahrens ist ein Ätzen und/oder eine Strukturierung ausgesucht aus der Gruppe umfassend Trockenätzen, nasschemisches Ätzen und/oder Plasmaätzen, insbesondere Reaktives Ionenätzen, Reaktives Ionentiefenätzen (Bosch-Prozess); und/oder das Abscheiden ausgesucht aus der Gruppe umfassend physikalische Gasphasenabscheidung (PVD), insbesondere thermisches Verdampfen, Laserstrahlverdampfen, Lichtbogenverdampfen, Molekularstrahlepitaxie, Sputtern, chemische Gasphasenabscheidung (CVD) und/oder Atomlagenabscheidung (ALD).

Diese Verfahren sind für die Herstellung von feinen Strukturen mit Größenordnungen im Mikrometerbereich besonders geeignet. Insbesondere durch den Boschprozess können sehr feine Strukturen mit hohem Aspektverhältnis erzeugt werden, die für ein kompaktes, effizientes und integrierten Aufbau der Bauelemente des IR-Emitters von Vorteil sind.

In einem weiteren Aspekt betrifft die Erfindung ein System umfassend
a) einen modulierbaren Infrarot-Emitter gemäß der Erfindung oder bevorzugten Ausführungsformen davon
b) eine Steuereinrichtung,
wobei die Steuereinrichtung konfiguriert ist zur Regulation des Aktuators für eine Relativbewegung des Deckelementes und des Grundelementes zwischen einer ersten und zweiten Position, um die Intensität der Emission des Infrarot-Emitters zu modulieren.

Die Steuereinrichtung ermöglicht bevorzugt eine Eingabe und setzt diese Eingabe in geeignete Steuersignale um. Eine Eingabe kann beispielsweise ein gewünschtes Spektrum, eine gewünschte Resonanzwellenlänge, eine gewünschte Intensität, Modulationstiefe und/oder Modulationsfrequenz sein. Die Steuereinrichtung erzeugt in erster Linie entsprechende analoge elektrische Signale, welche an den Aktuator und/oder das Mikro-Heizelement weitergegeben werden, um die gewünschte IR-Strahlung zu erzeugen.

Es können aber auch komplexere Signale als Eingabe dienen, welche einen genauen zeitlichen Amplitudenverlauf der ausgehenden IR-Strahlung für ein gewünschtes Spektrum vorgeben. Die Steuereinrichtung sorgt dann ebenfalls bevorzugt für die geeigneten Steuersignale zur Erzeugung der gewünschten, modulierten IR-Strahlung.

Die Steuereinrichtung ist insbesondere konfiguriert für eine Regulation des Aktuators für die Relativbewegung zwischen Grundelement und Deckelement zwischen (mindestens) einer ersten und (mindestens) einer zweiten Position. Dafür werden elektrische Signale erzeugt, die die erforderliche Translationsbewegung des mindestens einen Aktuators auslösen.

Bevorzugt umfasst die Steuereinrichtung eine Regelschleife, wobei durch einen Feedbackmechanismus eine Diskrepanz zwischen gewünschter Steuerung und tatsächlicher Bewegung des Aktuators und/oder Erhitzung des Heizelements korrigiert werden kann.

Es kann bevorzugt sein, dass ebenfalls der Temperaturverlauf des Heizelements zur zusätzlichen, langsamen Modulation der IR-Strahlung durch die Steuereinrichtung geregelt werden kann.

Die Steuereinrichtung des Systems kann sowohl extern als auch auf dem IR-Emitter integriert vorliegen.

Die Steuereinrichtung umfasst bevorzugt einen Prozessor, beispielsweise einen Mikroprozessor. Es können auch andere integrierte Schaltungen, welche in der digitalen Elektronik zur Steuerung verwendet werden, zum Einsatz kommen.

Die Verwendung eines solchen Systems umfassend eine geeignete Steuereinrichtung kann die gewünschte Verwendung des IR-Emitters erheblich vereinfachen. Beispielsweise können geeignete Spektroskopiesignale am PC gestaltet werden. Über die Eingabe werden die gewünschten Signale an die Steuereinrichtung übertragen. Von dieser werden die Ansteuerungssignale erzeugt, welche ein entsprechendes IR-Signal in hoher Übereinstimmung mit den theoretischen Vorgaben sicherstellt.

Eine Steuereinrichtung, insbesondere in Form einer in den Emitter integrierten Steuerung, ist sehr kompakt und einfach zu handhaben. Die Steuereinrichtung weist für die Eingabe bevorzugt eine geeignete Schnittstelle zur Verbindung bspw. mit einem Computer auf. Es kann ebenso gewünscht sein, dass über diese Schnittstelle auch Daten von der Steuerung an das Eingabegerät übertragbar sind, wie beispielsweise die aktuelle Temperatur des Heizelements, die Modulationsfrequenz oder andere Statusinformationen.

In einer weiteren bevorzugten Ausführungsform des Systems ist die Steuereinrichtung konfiguriert zur Regulation der Temperatur der erhitzbaren Bereiche des Mikro-Heizelements, bevorzugt in einem Bereich zwischen 50 °C und 1000 °C.

Eine solche Steuereinrichtung ist bevorzugt in der Lage, dem Heizelement geeignete elektrische Leistungen zur Verfügung zu stellen. Insbesondere soll die Temperatur hinreichend genau einstellbar sein und/oder konstant gehalten werden können. Dafür kann ein Regelmechanismus mit einer Feedbackschleife verwendet werden. Zur Messung der aktuellen Temperatur des Heizelements kann z. B. mindestens ein Temperatursensor an geeigneter Stelle des Elements integriert sein.

In einer weiteren bevorzugten Ausführungsform des Systems ist die Steuereinrichtung konfiguriert, den Aktuator für eine oszillierende Relativbewegung des Deckelementes und des Grundelementes zwischen einer ersten und einer zweiten Position zu regulieren, wobei bevorzugt eine Modulationsfrequenz der emittierten Infrarotstrahlung zwischen 10 Hz und 100 kHz erreicht wird.

Bevorzugt ist, dass die vom Aktuator ausgelöste Translationsbewegung zwischen einer ersten und einer zweiten Position regelmäßig wiederholt wird, so dass es zu einer Oszillation zwischen den Positionen kommt und die Translationsbewegung eine Periodizität aufweist. Dabei soll am Ende der Translationsbewegung bevorzugt wieder der Anfangspunkt der Bewegung erreicht werden und die Bewegung in der darauffolgenden Periode von Neuem ausgeführt werden. Dabei legt die Frequenz der oszillierenden Bewegung bevorzugt die resultierende Modulationsfrequenz der Leistungsintensität der emittierten Infrarotstrahlung fest.

Es kann auch eine im Rahmen der elektronischen Auflösung und/oder Bandbreite der Steuerungseinrichtung und/oder des Aktuators stufenlose Einstellung der Translationsfrequenz und somit der Modulationsfrequenz vorgenommen werden. Somit kann die Modulationsfrequenz bevorzugt im zeitlichen Verlauf variiert werden.

Es kann des Weiteren bevorzugt sein, dass nicht nur die Translationsfrequenz, sondern auch die Translationsamplitude im Rahmen der Bewegungsmöglichkeiten des Aktuators variiert wird.

So kann beispielsweise die vertikalen Abstandsänderungen zwischen Deckelement und dielektrischer Zwischenschicht oder aber der Grad der Überdeckung von Deckelement und Grundelement variiert werden. Hierdurch kann der Emissionsgrad und mithin die modulationstiefe in den angefahrenen Positionen zeitlich variabel eingestellt werden.

Somit ergibt sich ein System, welches sehr flexibel und effizient eine Variation der Modulationsfrequenz und Modulationstiefe der IR-Strahlung ermöglicht.

In einer weiteren bevorzugten Ausführungsform des Systems ist die Steuereinrichtung konfiguriert, den Aktuator für eine oszillierende Relativbewegung des Grundelementes und des Deckelementes derart zu regulieren, dass eine Modulationsfrequenz der emittierten Infrarotstrahlung zwischen 10 Hz und 100 kHz, besonders bevorzugt zwischen 100 Hz und 20 kHz, erreicht wird.

Hierfür ist besonders bevorzugt, dass alle benötigten Bauteile, wie Steuerungseinrichtung, Aktuator etc., die benötigte Bandbreite ermöglichen.

Die genannten Frequenzen haben sich für die bevorzugten Anwendungen im Bereich der Spektroskopie als besonders wirksam erwiesen. Insbesondere zur Verwendung in der photoakustischen Spektroskopie haben sich diese Frequenzen als besonders geeignet erwiesen, da sie einen großen Bereich akustischer Frequenzen abdecken, deren Erzeugung bei dieser Spektroskopiemethode im Vordergrund steht.

Ein Beispiel eines Verfahrens zur modulierten Emission von Infrarotstrahlung umfasst folgende Schritte:
- Bereitstellen eines modulierbaren Infrarot-Emitters gemäß der vorhergehenden Beschreibung;
- Erhitzen des Heizelementes zur Emission einer Infrarotstrahlung;
- Steuerung des Aktuators für eine Relativbewegung des Deckelementes und des Grundelementes zwischen einer ersten und zweiten Position, um die Intensität der Emission des Infrarot-Emitters zu modulieren.

Der durchschnittliche Fachmann erkennt, dass technische Merkmale, Definitionen und Vorteile bevorzugter Ausführungsformen des erfindungsgemäßen IR-Emitters und des Systems auch für das Verfahren gelten.

Beispiele der Verwendung eines modulierbaren Infrarot-Emitters gemäß der vorhergehenden Beschreibung oder eines Systems gemäß der vorhergehenden Beschreibung sind die photoakustische Spektroskopie und/oder die Infrarotspektroskopie.

Ein kompakter, langlebiger IR-Emitter und der mit hoher Geschwindigkeit modulierbar ist, ist für eine Vielzahl von Anwendungen interessant. Gerade für die photoakustische Spektroskopie sind viele Anwendungen denkbar, die nicht im Labor stattfinden und unter Alltagsbedingungen funktionieren müssen. Zu nennen sind beispielsweise das Gebiet der Umweltsensorik zur Detektion von (Schad-) Stoffen in der Umgebungsluft oder als Warnmelder für giftige Gase, wie beispielsweise Kohlenmonoxid.

Durch die hohen Modulationsfrequenzen von einigen kHz können gegenüber einer direkten elektrischen Modulation des Heizelements deutlich verbesserte Signal-zu-Rauschverhältnisse erzielt werden, wodurch belastbare Messungen möglich werden.

In einem weiteren Aspekt betrifft die Erfindung ein Photoakustisches Spektroskop zur Analyse von Gas, umfassend
- einen modulierbaren Infrarot-Emitter gemäß der vorstehenden Beschreibung,
- ein mit Gas befüllbares Analysevolumen,
- einen Schalldetektor,
wobei das Analysevolumen zwischen dem Infrarot-Emitter und dem Schalldetektor angeordnet ist, sodass die von dem Infrarot-Emitter modulierbar emittierte Infrarotstrahlung zur photoakustischen Spektroskopie des Gases genutzt werden kann.

Dem Fachmann ist bekannt, wie die photoakustische Spektroskopie durchgeführt wird und welche Komponenten dabei Verwendung finden. Der modulierbaren Infrarot-Emitter ist dabei bevorzugt so angeordnet und konfiguriert, dass von dem Infrarot-Emitter emittierte Infrarotstrahlung auf das Gas im Analysevolumen trifft. Erfolgt die modulierte Bestrahlung mit einer Infrarotwellenlänge welche dem Absorptionsspektrum eines in dem Gas befindlichen Moleküls entspricht, findet eine modulierte Absorption statt, die zu Erwärmungs- und Abkühlungsprozessen führt, deren Zeitskalen die Modulationsfrequenz der Strahlung widerspiegeln. Gemäß des photoakustischen Effekts führen die Erwärmungs- und Abkühlungsprozesse führen zu Expansionen und Kontraktion des Gases, wodurch Schallwellen mit der Modulationsfrequenz verursacht werden. Diese lassen dann sich durch den Schalldetektor messen. Die Leistung der Schallwellen ist dabei vorzugsweise direkt proportional zur Konzentration des absorbierenden Gases. Bevorzugt kann der Schalldetektor bzw. Schalldruckdetektor ein Mikrofon sein. Ebenso können aber auch sonstige passiv durch die Schallwellen in Schwingung versetzbare Strukturen, wie z. B. Membranen oder auch Schwingungsbalken als Schalldetektoren verwandt werden.

Das zu analysierende Gas befindet sich dabei in einem mit Gas befüllbaren Analysevolumen. In einer bevorzugten Ausführungsform ist dieses ein nach außen zumindest bereichsweise abgeschlossenes oder abschließbares Volumen (bzw. Kammer), in der sich das Gas befindet bzw. eingeleitet werden kann, z. B. durch eine verschließbare Öffnung in Form eines Verschlusses und/oder Ventils und/oder durch eine Zuleitung. Es kann sich aber auch um ein komplett abgeschlossenes oder abschließbares Volumen bzw. Kammer handeln, welches zumindest eine, vorzugsweise zwei verschließbare Öffnung zur Einleitung und/oder Ausleitung des zu analysierenden Gases aufweist. So kann das zu analysierende Gas sehr gut lokalisiert werden, insbesondere in einem Strahlbereich der Infrarotstrahlung.

Das Analysevolumen kann vorzugsweise ebenso zumindest teilweise geöffnet sein. Dadurch kann insbesondere eine das Spektroskop umgebende Gasatmosphäre, gegenüber dem das Analysevolumen zumindest teilweise geöffnet ist, vermessen und auf ihre Zusammensetzung überprüft werden. Dies ist insbesondere interessant für Anwendungen im Bereich der Schadstoffmessung, aber auch z. B. für militärische Anwendungen oder zur Terrorabwehr, z. B. durch einen Giftgasangriff.

Vorteilhaft ist in diesem Fall, dass das Analysevolumen wohl definiert ist, so dass Infrarot-Emitter, das Analysevolumen und der Schalldetektordass derart angeordnet vorliegen, dass die von dem Infrarot-Emitter modulierbar emittierbare Infrarotstrahlung Gas im Analysevolumen zur Ausbildung von Schalldruckwellen anregen kann, welche mit Hilfe des Schalldruckdetektors messbar sind.

Das Analysevolumen liegt bevorzugt im Strahlengang des Infrarot-Emitters vor. Das bedeutet bevorzugt, dass die Intensität des Strahls im Wesentlichen oder zumindest teilweise auf die dem Emitter zugewandte Seite des Analysevolumens trifft. Teilweise bedeutet bevorzugt zu mindestens 40 %, bevorzugt mindestens 50%, 60%, 70%, 80% oder mehr.

In einer bevorzugten Ausführungsform kann der Infrarot-Emitter von außen auf einen Vorzugsbereich des Analysevolumens ausgerichtet werden Muss dabei durch eine Außenwand des Volumens hindurch gestrahlt werden, um ein Gas im Inneren anzuregen, ist die Außenwand bevorzugt zumindest in diesem Bereich für die IR-Strahlung im Wesentlichen transparent. Der Infrarot-Emitter kann aber auch im Inneren des Analysevolumen angeordnet vorliegen.

Das Analysevolumen ist bevorzugt mit Gas befüllbar. Das bedeutet, dass auch bei einem zumindest teilweise abgeschlossenen bzw. verschließbaren Volumen eine vorzugsweise verschließbare Öffnung zur Befüllung vorliegt. Vorzugsweise kann auch ein Gasfluss realisiert werden, indem das Analysevolumen z. B. einen Zulauf und einen Ablauf besitzt. Somit kann entweder ein kontinuierlicher Gasfluss in das Volumen realisiert werden oder ein diskontinuierlicher Gasfluss, bei dem z. B. während einer Befüllungs- bzw. Gasaustauschphase eine Befüllung bzw. ein Austausch des Gases im Analysevolumen vorgenommen werden kann. In einer Analysephase wird der Gasfluss vorzugsweise unterbrochen, damit die photoakustische Spektroskopie stattfinden kann. Sollen zu verschiedenen Zeitpunkten verschiedene Gase analysiert werden, kann dies vorzugsweise durch einen solchen Aufbau realisiert werden. Zwischen zwei zu analysierenden Gasen kann vorzugsweise ein Reinigungsgas zur Reinigung des Volumens von etwaigen Gasrückständen zugeführt werden.

Bei einem Analysevolumen, welches zumindest teilweise geöffnet ist und somit vorzugsweise einen permanenten Gasaustausch mit einer Umgebung zulässt, findet eine Befüllung des Analysevolumens durch Wechselwirkung mit einer Gasatmosphäre der Umgebung statt.

Ein Analysevolumen kann vorzugsweise ein Probekammer und eine Referenzkammer umfassen, welche durch einen Verbindungskanal verbunden bzw. verbindbar sind.

Im Falle einer Ausführungsform eines Analysevolumens, welche eine Probekammer und eine Referenzkammer aufweist, kann es bevorzugt sein, in jeder Kammer mindestens einen Schalldetektor aufzuweisen, um in jeder Kammer separat zu messen und somit Störquellen, z. B. externe Schalldruckwellen, welche nicht von der in der Probekammer absorbierten IR-Strahlung herrühren, vorzugsweise nach der Messung herausrechnen zu können.

Ebenso kann es bevorzugt sein, dass der Infrarotemitter die Probekammer und nicht die Referenzkammer bestrahlt und wobei zwischen der Probekammer und Referenzkammer ein Verbindungskanal vorliegt, in welchem sich ein Schalldetektor befindet. Diese Ausführungsform zeichnet sich durch eine besonders präzise photoakustische Spektroskopie aus, da beispielsweise Schall von unerwünschten Schallquellen bei der Messung und/oder der Auswertung der Messung herausgerechnet bzw. nicht mitgemessen wird. Vorzugsweise können Probenvolumen und ein Referenzvolumen im Wesentlichen gleiche Abmessungen aufweisen, um eine genaue differentielle Messmethode zu realisieren.

In dem Probenvolumen und Referenzvolumen kann sich das gleiche Gas befinden. Es kann ebenso bevorzugt sein, dass im Probevolumen und im Referenzvolumen unterschiedliches Gas umfasst ist, wobei im Referenzvolumen ein Gas mit bekannten Eigenschaften vorliegt und im Probenvolumen ein zu analysierendes Gas vorliegt. Durch die Verwendung zweier Volumina und mindestens eines Schalldruckdetektors kann vorteilhafterweise eine verbesserte Eliminierung von Fehlerquellen, z. B. unerwünschten Schallwellen erfolgen, weil diese auf beide Volumina wirken und der zwischen den Volumina angeordnete Schalldruckdetektor bevorzugt im Wesentlichen nur die durch die für die Photoakustische Spektroskopie relevanten von der IR-Strahlung hervorgerufenen Schalldruckwellen im Probenvolumen als Differenzsignal zwischen Probenvolumen und Referenzvolumen detektiert.

Aufgrund des aus dem Stand der Technik nicht bekannten, kompakten und hochfrequent modulierbaren IR-Emitters kann der ganze Aufbau für verschiedene Varianten eines photoakustischen Spektroskops stets besonders kompakt und alltagstauglich hergestellt werden. Durch die hohen Modulationsfrequenzen sind die Analysemöglichkeiten zudem äußerst vielfältig, wobei insbesondere das Signal-zu-Rauschverhältnis erhöht werden kann. Ein typisches 1/f Rauschen bei Schalldetektoren, wie Mikrofonen, kann so beispielsweise deutlich verringert werden.

In einer bevorzugten Ausführungsform umfasst das photoakustische Spektroskop mindestens zwei oder mehr modulierbare Infrarot-Emitter gemäß der vorstehenden Beschreibung, wobei sich die Resonanzwellenlängen der zwei oder mehr modulierbarer Infrarot-Emitter unterscheiden. Wie vorstehend ausgeführt, kann insbesondere durch eine geeignete Strukturierung des Metamaterials bzw. der sich periodisch wiederholenden Einheitszellen die Resonanzwellenlänge eingestellt werden, bei welcher in der zweiten Position eine hohe Emission der Infrarotstrahlung erfolgt.

Durch Verwendung von zwei oder mehr modulierbaren Infrarot-Emittern mit unterschiedlichen Resonanzwellenlängen können somit unterschiedliche Moleküle in dem Gas gleichzeitig analysiert werden. Da die oder zwei oder mehr modulierbaren Infrarot-Emitter auch über separate Aktuatoren verfügen, können diese auch bevorzugt mittels unterschiedlicher Modulationsfrequenzen betrieben werden, wodurch sich die Schallwellen der unterschiedlichen Moleküle besonders präzise getrennt detektieren lassen. Aufgrund der kompakten Bauweise kann somit ein miniaturisiertes photoakustisches Spektroskop bereitgestellt werden, welches sich durch eine hohe Bandbreite an gleichzeitig detektierbaren Molekülen, bei höchster Genauigkeit auszeichnet.

### Detaillierte Beschreibung

Im Folgenden soll die Erfindung an Hand von Beispielen und Figuren näher erläutert werden, ohne auf diese beschränkt zu sein.

### Kurzbeschreibung der Abbildungen

**Figur 1** zeigt eine schematische Darstellung eines Querschnittes einer bevorzugten Ausführungsform des modulierbaren Infrarot-Emitters.
**Figur 2** zeigt eine schematische 3D-Ansicht einer bevorzugten Ausführungsform des modulierbaren Infrarot-Emitters.

### Detaillierte Beschreibung der Abbildung

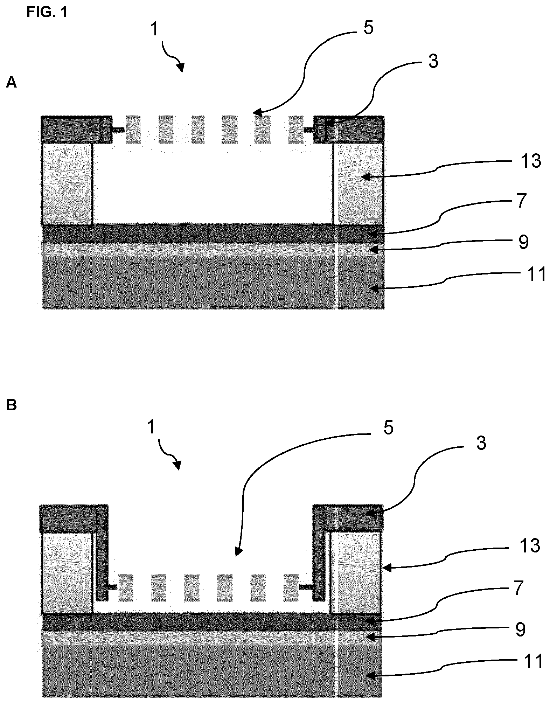

Figur 1 zeigt eine schematische Darstellung einer bevorzugten Ausführungsform eines modulierbaren Infrarot-Emitters **1** im Querschnitt. Auf einem Heizelement 11 liegt ein Schichtaufbau umfassend sich ein flächiges Grundelement **9,** eine dielektrische Zwischenschicht **7** und ein flächiges Deckelement **5** aus einem strukturierten Metamaterial vor. Das Heizelement 11 kann beispielsweise ein Si-Substrat umfassen, auf welchem eine erhitzbare Schicht aus einem leitfähigen Material aufgebracht ist, an welchem Kontakte für eine Strom- und/oder Spannungsquelle vorliegen. Es kann aber auch bevorzugt sein, dass das Grundelement **9** als eine erhitzbare Schicht fungiert und zu diesem Zweck kontaktiert wird. Das Grundelement **9** und das Deckelement **5** sind bevorzugt aus einem leitfähigen Material, besonders bevorzugt aus einem Metall gefertigt.

Das Deckelement **5** ist mit Aktuatoren **3** gekoppelt, welche für eine vertikale Translationsbewegung des Deckelements **5** gegenüber dem Grundelement konfiguriert sind und mindestens zwei Positionen ansteuern können.

In einer ersten Position (Fig. 1A) wird das Deckelement **5** in einem größeren Abstand oberhalb der dielektrischen Zwischenschicht **7** gehalten, als in einer zweiten Position (Fig. 1B).

Zu diesem Zweck liegt in der gezeigten Ausführungsform ein Abstandsrahmen **13** auf der dielektrischen Zwischenschicht **7** vor, welcher den vertikalen Abstand des Deckelementes **5** zur dielektrischen Zwischenschicht **7** in der ersten Position definiert. Der Abstandsrahmen **13** kann beispielsweise eine Nitrid oder Oxidschicht sein, welche entlang der Umrandung der dielektrischen Zwischenschicht **7** mit einer definierten Höhe aufgetragen ist. Die Aktuatoren **3** sind sowohl mit dem Abstandsrahmen **13,** als auch mit dem Deckelement **5** gekoppelt und dafür konfiguriert, in einer ersten Position das Deckelement **5** auf Höhe des oberen Endes des Abstandsrahmens **13** zu halten, während in der zweiten Position ein Absenken des Deckelement **5** zur dielektrischen Zwischenschicht **7** hin, bevorzugt bis zur Kontaktierung, erfolgt.

Die Aktuatoren **3** sind bevorzugt MEMS-Aktuatoren, besonders bevorzugt elektrostatische MEMS-Aktuatoren, wobei beispielsweise durch Anlegung einer Spannung das Deckelement **5** gezielt in eine erste oder zweite Position gefahren werden kann.

Durch die mittels der Aktuatoren **3** gesteuerte Translationsbewegung des Deckelementes **5** erfolgt eine Modulation der Intensität des Infrarot-Emitters **1.**

Während des Betriebes des Infrarot-Emitters **1** wird das Heizelement **11** bevorzugt auf eine Temperatur in einem Bereich von 50° bis 1000° gesteuert. Die resultierende emittierte Infrarotstrahlung hängt davon ab, in welchem Abstand sich das Deckelement **5** aus einem strukturierten Metamaterial oberhalb der dielektrischen Zwischenschicht **7** bzw. dem Grundelement **9** befindet.

In der in Figur 1 B gezeigt zweiten Position befindet sich das Deckelement **5** in einem Abstand von bevorzugt weniger als 200 nm, besonders bevorzugt in einem Abstand, welcher einem Kontakt der beiden Elemente entspricht. In dem Zustand bilden das Deckelement **5** aus einem strukturierten Metamaterial, die dielektrische Zwischenschicht **7** und das Grundelement **9** auf dem Heizelement **11** bevorzugt einen *metamaterial perfect absorber,* wodurch für eine oder mehrere bevorzugte Resonanzwellenlängen ein besonders hoher Emissionsgerad erreicht wird. In anderen Worten tritt in der zweiten Position eine elektromagnetisch Resonanz auf, welche die Ankopplung der Infrarotstrahlung an eine bestimmte Resonanzwellenlänge erlaubt.

Der vertikale Abstand in der ersten Position (Figur 1A) ist derart gewählt, dass keine resonante Kopplung auftreten kann und der Infrarot-Emitter **1** einen geringen Emissionsgrad aufweist.

Der durch die Aktuatoren **3** vermittelte Wechsel zwischen einer ersten (nicht-resonanten) und einer zweiten (resonanten) Position moduliert somit die Intensität der emittierten Infrarotstrahlung. Vereinfacht ausgedrückt ist der IR-Emitter **1** in der ersten Position "aus" und in der zweiten Position "an". Durch MEMS-Aktuatoren kann vorteilhafterweise eine hochfrequente Modulation zwischen den beiden Zuständen im kHz-Bereich erzielt werden, wodurch sich der beschriebenen Infrarot-Emitter insbesondere für Anwendungen in der Infrarotspektroskopie eignet.

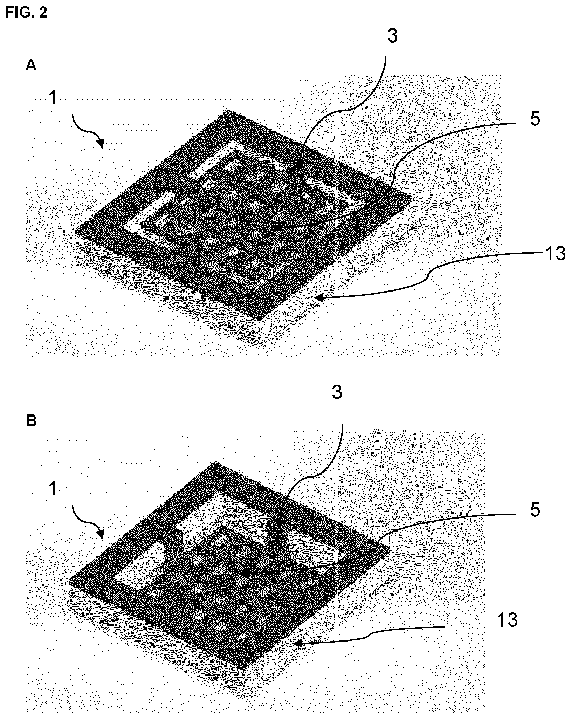

**Figur 2** zeigt eine schematische 3D-Ansicht von oben auf eine bevorzugte Ausführungsform eines modulierbaren Infrarot-Emitters **1.** Bevorzugt handelt sich um eine Ausführungsform eines modulierbaren Infrarot-Emitter **1,** wie er in Bezug auf den in Fig. 1 gezeigten Querschnitt, erläutert wurde. Wie in der 3D-Ansicht ersichtlich, umfasst der bevorzugte Infrarot-Emitter **1** vier MEMS-Aktuatoren **3,** welche an den äußeren Seiten des Deckelementes **5** installiert vorliegen. Bevorzugt sind die vier MEMS-Aktuatoren **3** jeweils an den Abstandsrahmen 13 sowie an das Deckelement **5** gekoppelt und für eine vertikale Relativbewegung des Deckelementes **5** von einer ersten Position (Fig. 2A) zu einer zweiten Position (Fig. 2B) konfiguriert. Die Verwendung der vier MEMS-Aktuatoren **3** erlaubt ein besonders schnelles und zuverlässiges Absenken des Deckelementes **5** auf einen gewünschten Abstand.

Wie im Hinblick auf Fig. 1 erläutert, erfolgt durch das vertikale Absenken des Deckelementes **5** aus strukturiertem Metamaterial auf die (nicht gezeigte) dielektrische Zwischenschicht eine resonante Abstrahlung der Infrarotstrahlung bei einer oder mehrere bevorzugter Resonanzwellenlängen. In der schematischen Abbildung ist die Strukturierung des Metamaterials durch rechteckige Einheitszellen angezeigt. Diese dienen vorwiegend der Illustration und es können, wie vorgehend beschriebenen, verschiedene Formen und/oder Dimensionierungen der Einheitszellen verwandt werden, um eine effektive Resonanzabstrahlung in der zweiten Position, zu gewährleisten.

### Bezugszeichenliste

- 1: modulierbarer Infrarot-Emitter
- 3: Aktuator
- 5: Deckelement aus strukturiertem Metamaterial
- 7: dielektrische Zwischenschicht
- 9: Grundelement aus einem leitfähigen Material
- 11: Heizelement
- 13: Abstandsrahmen

### REFERENZEN

[1] Liu et al.: Micromachined tunable metamaterials: a review, Journal of Optics 14, 114009, 2012.
[2] Zheludev et al.: From metamaterials to metadevices, Nature Materials , Vol 11, 2012.
[3] Tao et al.: MEMS Based Structurally Tunable Metamaterials at Terahertz Frequencies, J Infrared Milli Terahz Waves Vol 32, 580-595, 2011.
[4] Turpin et al.: Reconfigurable and Tunable Metamaterials: A Review of the Theory and Applications, International Journal of Antennas and Propagation,Volume 2014, Article ID 429837, 2014.
[5] Hildenbrand et al.: Micromachined Mid-Infrared Emitter for Fast Transient Temperature Operation for Optical Gas Sensing Systems. IEEE Sensors Journal, 10 (2), 2010.
[6] Spannhake et al.: High-temperature MEMS Heater Platforms: Long-term Performance of Metal and Semiconductor Heater Materials, Sensors 2006, 6, 405-419.
[7] Harald A. Beck, Anwendung der Photoakustischen Spektroskopie in der Prozess- und Umweltanalytik, Dissertation, TU München, 2003.
[8] Pusch et al.: A highly efficient CMOS nanoplasmonic crystal enhanced slow-wave thermal emitter improves infrared gas-sensing devices, Nature Scientific Reports, 5:17451, 2015.
[9] Lochbaum et al.: On-Chip Narrowband Thermal Emitter for Mid-IR Optical Gas Sensing, ACS Photonics, 4, 1371-1380, 2017.
[10] Tsai, et al.: Reflection and emission properties of an infrared emitter, Optics Express, Vol. 15, No. 22, 2007.
[11] V. G. Veselago, Sov. Phys. Usp. 1968, 10 , 509.
[12] J. B. Pendry , A. J. Holden , D. J. Robbins , W. J. Stewart , IEEE Trans. Microwave Theory Tech. 1999 , 47 , 2075.
[13] N. I. Landy , S. Sajuyigbe , J. J. Mock , D. R. Smith , W. J. Padilla , Phys. Rev. Lett. 2008 , 100 , 207402.
[14] X. Liu , T. Starr , A. F. Starr , W. J. Padilla , Phys. Rev. Lett. 2010 , 104 , 207403.
[15] Claire M. Watts , Xianliang Liu , and Willie J. Padilla. Metamaterial Electromagnetic Wave Absorbers, Adv. Mater. 2012. 24, OP98-OP120.
[16] I. Puscasu , W. L. Schaich , Appl. Phys. Lett. 2008 , 92 , 233102.
[17] Qu et al. Micromachines 2016, 7, 14; doi:10.3390/mi7010014.
[18] T. Inoue, M. de Zoysa, T. Asano und S. Noda, Realization of narrowband thermal emission with optical nanostructures, Optica vol. 2, no. 2015.
[19] X: Liu and W. J. Padilla, Reconfigurable room temperature metamatenial infrared emitter, Optica, vol. 4, no. 4, 2017.
[20] EP3315929
[21] WO2017088071
[22] US2012170114
[23] US2017288125.

## Patentansprüche

1. Photoakustisches Spektroskop zur Analyse von Gas, umfassend
- einen modulierbaren Infrarot-Emitter (1)
- ein mit Gas befüllbares Analysevolumen und
- einen Schalldetektor,
wobei das Analysevolumen zwischen dem Infrarot-Emitter (1) und dem Schalldetektor angeordnet ist, sodass die von dem Infrarot-Emitter (1) modulierbar emittierte Infrarotstrahlung zur photoakustischen Spektroskopie des Gases genutzt werden kann und das
**dadurch gekennzeichnet ist, dass**
der modulierbare Infrarot-Emitter (1)
- ein Heizelement (11)
- ein flächiges Grundelement (9) aus einem leitfähigen Material
- eine dielektrische Zwischenschicht (7)
- ein flächiges Deckelement (5) aus einem leitfähigen Material und
- ein Aktuator (3)
umfasst, wobei das Deckelement (5) ein strukturiertes Metamaterial ist mit periodisch angeordneten Einheitszellen und der Aktuator (3) konfiguriert ist für eine Relativbewegung des Deckelementes (5) und des Grundelementes (9) zwischen einer ersten und zweiten Position, um die Intensität der Emission des Infrarot-Emitters (1) zu modulieren.

2. Photoakustisches Spektroskop gemäß dem vorherigen Anspruch,
**dadurch gekennzeichnet, dass**
die Relativbewegung eine vertikale Translationsbewegung des Deckelementes (5) und/oder Grundelementes (9) entlang der Emissionsrichtung des Infrarot-Emitters (1) umfasst, welche den Abstand zwischen dem Deckelement (5) und dem Grundelement (9) ändert.

3. Photoakustisches Spektroskop gemäß einem der vorherigen Ansprüche,
**dadurch gekennzeichnet, dass**
die Relativbewegung eine horizontale Translationsbewegung des Deckelementes (5) und/oder des Grundelementes (9) orthogonal zur Emissionsrichtung des Infrarot-Emitters (1) umfasst, welche den Grad der Überdeckung zwischen dem Deckelement (5) und dem Grundelement (9) ändert.

4. Photoakustisches Spektroskop gemäß einem oder mehreren der vorherigen Ansprüche
**dadurch gekennzeichnet, dass**
der Emissionsgrad in Richtung der Flächennormale des Deckelementes (5) für mindestens eine Resonanzwellenlänge in einem Bereich von 1 µm bis 10 µm in der zweiten Position um einen Faktor 2, bevorzugt 4, mehr bevorzugt 8 höher ist als in der ersten Position, wobei es bevorzugt ist, dass der Emissionsgrad in Richtung der Flächennormale des Deckelementes (5) für mindestens eine Resonanzwellenlänge in einem Bereich von 1 µm bis 10 µm in der zweiten Position einen Wert von mehr als 0.7, bevorzugt mehr als 0,8, 0,9 und in der ersten Position einen Wert von weniger als 0,4, bevorzugt weniger als 0,3, 0,2 aufweist.

5. Photoakustisches Spektroskop gemäß einem oder mehreren der vorherigen Ansprüche
**dadurch gekennzeichnet, dass**
das Deckelement (5) aus einem Metall, besonders bevorzugt aus Gold, Silber, Aluminium, Wolfram, Molbidän, Titan und/ oder Kupfer gefertigt ist und/oder das Grundelement (9) eine kontinuierliche leitfähige Schicht ist und bevorzugt aus demselben leitfähigen Material gefertigt ist wie das Deckelement (5).

6. Photoakustisches Spektroskop gemäß einem oder mehreren der vorherigen Ansprüche
**dadurch gekennzeichnet, dass**
die dielektrische Zwischenschicht (7) aus einem Material gefertigt ist ausgewählt aus einer Gruppe umfassend Aluminiumnitrid, Siliziumnitrid, Aluminiumoxid, Siliziumoxid, Titandioxid und/oder Tantaloxid.

7. Photoakustisches Spektroskop gemäß einem oder mehreren der vorherigen Ansprüche,
**dadurch gekennzeichnet, dass**
der Infrarot-Emitter (1) mindestens vier MEMS-Aktuatoren umfasst, welche an äußeren Seiten des Deckelementes (5) installiert vorliegen und konfiguriert sind die Relativbewegung des Deckelementes (5) und des Grundelementes (9) zwischen der ersten und zweiten Position simultan zu steuern.

8. Photoakustisches Spektroskop gemäß einem oder mehreren der vorherigen Ansprüche,
**dadurch gekennzeichnet, dass**
in der ersten Position das Deckelement (5) einen Abstand zur dielektrischen Schicht von mindestens 500 nm, bevorzugt mindestens 1000 nm aufweist und in der zweiten Position einen Abstand zur dielektrischen Schicht von höchstens 200 nm, bevorzugt höchstens 50 nm, besonders bevorzugt 0 µm aufweist.

9. Photoakustisches Spektroskop gemäß einem oder mehreren der vorherigen Ansprüche sofern abhängig vom Anspruch 3,
**dadurch gekennzeichnet, dass**
in der ersten Position das Deckelement (5) und Grundelement (9) einen Grad der Überdeckung von weniger als 40%, bevorzugt weniger als 10% haben und in der zweiten Position das Deckelement (5) und Grundelement (9) einen Grad der Überdeckung von mehr als 40%, bevorzugt mehr als 10% haben, wobei der vertikale Abstand zwischen Deckelement (5) und Grundelement (9) in der zweiten Position bevorzugt weniger als 200 nm beträgt.

10. Photoakustisches Spektroskop gemäß einem oder mehreren der vorherigen Ansprüche
**dadurch gekennzeichnet, dass**
das photoakustische Spektroskop eine Steuereinrichtung umfasst, wobei die Steuereinrichtung konfiguriert ist zur Regulation des Aktuators für eine Relativbewegung des Deckelementes (5) und des Grundelementes (9) zwischen einer ersten und zweiten Position, um die Intensität der Emission des Infrarot-Emitters (1) zu modulieren.

11. Photoakustisches Spektroskop gemäß einem oder mehreren der vorherigen Ansprüche
**dadurch gekennzeichnet, dass**
die Steuereinrichtung konfiguriert ist den Aktuator (3) für eine oszillierende Relativbewegung des Deckelementes (5) und des Grundelementes (9) zwischen einer ersten und einer zweiten Position zu regulieren, wobei bevorzugt eine Modulationsfrequenz der Leistungsintensität der emittierten Infrarotstrahlung zwischen 10 Hz und 100 kHz erreicht wird.

12. Photoakustisches Spektroskop gemäß dem vorherigen Anspruch,
**dadurch gekennzeichnet, dass**
der Schalldetektor ein Mikrofon ist und die Modulationsfrequenz der Leistungsintensität der emittierten Infrarotstrahlung mindestens 1 kHz beträgt.

## Claims

1. Photoacoustic spectroscope for analyzing gas, comprising
- a modulatable infrared emitter (1)
- an analysis volume that can be filled with gas and
- a sound detector,
wherein the analysis volume is arranged between the infrared emitter (1) and the sound detector, so that the infrared radiation emitted in a modulatable manner by the infrared emitter (1) can be used for photoacoustic spectroscopy of the gas and which is
**characterized in that**
the modulatable infrared emitter (1) comprises
- a heating element (11)
- a planar base element (9) made of a conductive material
- a dielectric interlayer (7)
- a planar cover element (5) made of a conductive material and
- an actuator (3),
wherein the cover element (5) is a structured metamaterial with periodically arranged unit cells and the actuator (3) is configured for relative movement of the cover element (5) and the base element (9) between a first and second position in order to modulate the intensity of the emission of the infrared emitter (1).

2. Photoacoustic spectroscope according to the preceding claim
**characterized in that**
the relative movement comprises a vertical translational movement of the cover element (5) and/or base element (9) along the emission direction of the infrared emitter (1), which changes the distance between the cover element (5) and the base element (9).

3. Photoacoustic spectroscope according to one of the preceding claims
**characterized in that**
the relative movement comprises a horizontal translational movement of the cover element (5) and/or base element (9) orthogonal to the emission direction of the infrared emitter (1), which changes the degree of overlap between the cover element (5) and the base element (9).

4. Photoacoustic spectroscope according to one or more of the preceding claims
**characterized in that**
the emissivity in the direction of the surface normal of the cover element (5) for at least one resonance wavelength, in a range of from 1 µm to 10 µm, is higher in the second position than in the first position by a factor of 2, preferably 4, more preferably 8, wherein it is preferred that the emissivity in the direction of the surface normal of the cover element (5) for at least one resonance wavelength in a range from 1 µm to 10 µm in the second position has a value of more than 0.7, preferably more than 0.8, 0.9, and in the first position, a value of less than 0.4, preferably less than 0.3, 0.2.

5. Photoacoustic spectroscope according to one or more of the preceding claims
**characterized in that**
the cover element (5) is made of a metal, particularly preferably gold, silver, aluminum, tungsten, molybdenum, titanium, and/or copper, and/or
the base element (9) is a continuous conductive layer and is preferably made of the same conductive material as the cover element (5).

6. Photoacoustic spectroscope according to one or more of the preceding claims
**characterized in that**
the dielectric interlayer (7) is made of a material selected from the group consisting of aluminum nitride, silicon nitride, aluminum oxide, silicon oxide, titanium dioxide, and/or tantalum oxide.

7. Photoacoustic spectroscope according to one or more of the preceding claims
**characterized in that**
the infrared emitter (1) comprises at least four MEMS actuators, which are installed on the outer sides of the cover element (5) and are configured to control the relative movement of the cover element (5) and the base element (9) between the first and second position simultaneously.

8. Photoacoustic spectroscope according to one or more of the preceding claims
**characterized in that**
in the first position, the cover element (5) has a distance from the dielectric layer of at least 500 nm, preferably at least 1000 nm, and in the second position has a distance from the dielectric layer of at most 200 nm, preferably at most 50 nm, and particularly preferably 0 µm.

9. Photoacoustic spectroscope according to one or more of the preceding claims if dependent on claim 3,
**characterized in that**
in the first position, the cover element (5) and base element (9) have a degree of overlap of less than 40%, preferably less than 10%, and in the second position, the cover element (5) and base element (9) have a degree of overlap of more than 40%, preferably more than 10%, wherein the vertical distance between the cover element (5) and base element (9) in the second position is preferably less than 200 nm.

10. Photoacoustic spectroscope according to one or more of the preceding claims
**characterized in that**
the photoacoustic spectroscope comprises a control device, wherein the control device is configured to regulate the actuator for relative movement of the cover element (5) and the base element (9) between a first and second position in order to modulate the intensity of the emission of the infrared emitter (1).

11. Photoacoustic spectroscope according to one or more of the preceding claims
**characterized in that**
the control device is configured to regulate the actuator (3) for an oscillating relative movement of the cover element (5) and the base element (9) between a first and a second position, whereby a modulation frequency of the power intensity of the emitted infrared radiation is preferably achieved between 10 Hz and 100 kHz.

12. Photoacoustic spectroscope according to the previous claim
**characterized in that**
the sound detector is a microphone and the modulation frequency of the power intensity of the emitted infrared radiation is at least 1 kHz.

## Revendications

1. Spectroscope photoacoustique pour l'analyse des gaz, comprenant
- un émetteur infrarouge modulable (1)
- un volume d'analyse qui peut être rempli de gaz et
- un détecteur de son,
dans lequel le volume d'analyse est disposé entre l'émetteur infrarouge (1) et le détecteur sonore, de sorte que le rayonnement infrarouge émis par l'émetteur infrarouge (1) de manière modulable peut être utilisé pour la spectroscopie photoacoustique du gaz et le
**caractérisé en ce que**
l'émetteur infrarouge modulable (1) comprend
- un élément chauffant (11)
- un élément de base (9) plat constitué d'un matériau conducteur
- une couche intermédiaire diélectrique (7)
- un élément de recouvrement (5) plat constitué d'un matériau conducteur et
- un actionneur (3)
dans lequel l'élément de recouvrement (5) est un métamatériau structuré avec des cellules unitaires disposées périodiquement et l'actionneur (3) est configuré pour un mouvement relatif de l'élément de recouvrement (5) et de l'élément de base (9) entre une première et une seconde position afin de moduler l'intensité de l'émission de l'émetteur infrarouge (1).

2. Spectroscope photoacoustique selon la revendication précédente,
**caractérisé en ce que**
le mouvement relatif comprend un mouvement de translation vertical de l'élément de recouvrement (5) et/ou de l'élément de base (9) le long de la direction d'émission de l'émetteur infrarouge (1), ce qui modifie la distance entre l'élément de recouvrement (5) et l'élément de base (9).

3. Spectroscope photoacoustique selon l'une des revendications précédentes,
**caractérisé en ce que**
le mouvement relatif comprend un mouvement de translation horizontal de l'élément de recouvrement (5) et/ou de l'élément de base (9) perpendiculairement à la direction d'émission de l'émetteur infrarouge (1), ce qui modifie le degré de recouvrement entre l'élément de recouvrement (5) et l'élément de base (9).

4. Spectroscope photoacoustique selon l'une ou plusieurs des revendications précédentes,
**caractérisé en ce que**
l'émissivité dans la direction de la normale à la surface de l'élément de recouvrement (5) pour au moins une longueur d'onde de résonance dans une plage de 1 µm à 10 µm dans la seconde position est supérieure d'un facteur 2, de préférence 4, de manière davantage préférée 8, à celle dans la première position, dans lequel il est préférable que l'émissivité dans la direction de la normale à la surface de l'élément de recouvrement (5) pour au moins une longueur d'onde de résonance dans une plage de 1 µm à 10 µm, dans la seconde position, présente une valeur supérieure à 0,7, de préférence supérieure à 0,8 0,9 et, dans la première position, présente une valeur inférieure à 0,4, de préférence inférieure à 0,3 0,2.

5. Spectroscope photoacoustique selon une ou plusieurs des revendications précédentes
**caractérisé en ce que**
l'élément de recouvrement (5) est constitué d'un métal, de préférence d'or, d'argent, d'aluminium, de tungstène, de molybdène, de titane et/ou de cuivre et/ou
l'élément de base (9) est une couche conductrice continue et est de préférence constitué du même matériau conducteur que l'élément de recouvrement (5).

6. Spectroscope photoacoustique selon une ou plusieurs des revendications précédentes
**caractérisé en ce que**
la couche intermédiaire diélectrique (7) est constituée d'un matériau choisi dans un groupe comprenant le nitrure d'aluminium, le nitrure de silicium, l'oxyde d'aluminium, l'oxyde de silicium, le dioxyde de titane et/ou l'oxyde de tantale.

7. Spectroscope photoacoustique selon une ou plusieurs des revendications précédentes,
**caractérisé en ce que**
l'émetteur infrarouge (1) comprend au moins quatre actionneurs MEMS qui sont installés sur les côtés extérieurs de l'élément de recouvrement (5) et sont configurés pour contrôler simultanément le mouvement relatif de l'élément de recouvrement (5) et de l'élément de base (9) entre les première et seconde positions.

8. Spectroscope photoacoustique selon une ou plusieurs des revendications précédentes,
**caractérisé en ce que**
dans la première position, l'élément de recouvrement (5) présente une distance de la couche diélectrique d'au moins 500 nm, de préférence d'au moins 1000 nm, et dans la seconde position, une distance de la couche diélectrique d'au plus 200 nm, de préférence d'au plus 50 nm, de manière particulièrement préférée de 0 µm.

9. Spectroscope photoacoustique selon une ou plusieurs des revendications précédentes en fonction de la revendication 3,
**caractérisé en ce que**
dans la première position, l'élément de recouvrement (5) et l'élément de base (9) ont un degré de chevauchement inférieur à 40 %, de préférence inférieur à 10 %, et dans la seconde position, l'élément de recouvrement (5) et l'élément de base (9) ont un degré de chevauchement supérieur à 40 %, de préférence supérieur à 10 %, dans lequel la distance verticale entre l'élément de recouvrement (5) et l'élément de base (9) dans la seconde position est de préférence inférieure à 200 nm.

10. Spectroscope photoacoustique selon une ou plusieurs des revendications précédentes
**caractérisé en ce que**
le spectroscope photoacoustique comprend un dispositif de commande, dans lequel le dispositif de commande est configuré pour réguler l'actionneur pour un mouvement relatif de l'élément de recouvrement (5) et de l'élément de base (9) entre une première et une seconde position afin de moduler l'intensité de l'émission de l'émetteur infrarouge (1).

11. Spectroscope photoacoustique selon l'une ou plusieurs des revendications précédentes,
**caractérisé en ce que**
le dispositif de commande est configuré pour réguler l'actionneur (3) pour un mouvement relatif oscillant de l'élément de recouvrement (5) et de l'élément de base (9) entre une première et une seconde position, dans lequel, de préférence, une fréquence de modulation de l'intensité de puissance du rayonnement infrarouge émis comprise entre 10 Hz et 100 kHz est obtenue.

12. Capteur de gaz photoacoustique selon la revendication précédente,
**caractérisé en ce que**
le détecteur sonore est un microphone et la fréquence de modulation de l'intensité de puissance du rayonnement infrarouge émis est d'au moins 1 kHz.
